# EUROPÄISCHE PATENTSCHRIFT

(11) **EP 2 931 714 B1**
(45) Veröffentlichungstag und Bekanntmachung des Hinweises auf die Patenterteilung: **21.12.2016**
(21) Anmeldenummer: 13814824.2
(22) Anmeldetag: 14.11.2013
(51) Int. Cl.: C07D 309/40, C07D 405/12, A61K 8/49, A61Q 5/00, A61Q 5/06, C09B 43/00

(54) **SUBSTITUIERTE CHINONE ODER ANALOGA ALS FARBSTOFFE**
SUBSTITUTED QUINONES OR ANALOGUES AS COLOURING AGENTS
QUINONES SUBSTITUÉES OU ANALOGUES UTILISÉS COMME COLORANTS

(30) Priorität: 13.12.2012 EP 12008325
(43) Veröffentlichungstag der Anmeldung: 21.10.2015
(73) Patentinhaber: Merck Patent GmbH, 64293 Darmstadt (DE)
(72) Erfinder: RUDOLPH, Thomas, 64291 Darmstadt (DE); SCHEURICH, René Peter, 64846 Gross-Zimmern (DE)
(86) Internationale Anmeldenummer: PCT/EP2013/003430
(87) Internationale Veröffentlichungsnummer: WO 2014/090364

(56) Entgegenhaltungen:
- WO-A1-2012/028245
- WO-A1-2012/069476
- TÂNIA MONIZ ET AL: "Design of a water soluble 1,8-naphthalimide/3-hydroxy-4-pyridinone conjugate: Investigation of its spectroscopic properties at variable pH and in the presence of Fe3+, Cu2+ and Zn2+", DYES AND PIGMENTS, Bd. 98, Nr. 2, 13. Mai 2013 (2013-05-13), Seiten 201-211, XP055098984, ISSN: 0143-7208, DOI: 10.1016/j.dyepig.2013.02.020

## Beschreibung

Die Erfindung betrifft protein-adhäsive Farbstoffe, deren Herstellung und Zubereitungen, diese enthaltend sowie ein Verfahren zum Färben von keratinhaltigen Fasern, insbesondere zum Färben von menschlichen Haaren.

Derzeit ist zum Färben einer Matrix, wie zum Beispiel Haut, Haare, Nägel oder Textilien eine Vielzahl von Direktfarbstoffen bekannt. Dabei assoziieren sich die Farbstoffe an die Matrix und/oder bilden kovalente chemische Bindungen zu der Matrix aus. Diese Adhäsion und/oder Anbindung der Farbstoffmoleküle an die Matrix kann in unterschiedlicher Art und Weise stattfinden und zu einem unterschiedlichen Ergebnis hinsichtlich des Adhäsionscharakters führen. Deshalb zeichnen sich die Farbstoffe auch durch eine unterschiedlich starke Adhäsionsfähigkeit bis hin zu einer Anbindungsfähigkeit an die jeweilige Matrix aus.

Oft ist bei derzeit gebräuchlichen Farbstoffen eben diese Anbindungsfähigkeit schwach ausgeprägt, so dass der Farbstoff schnell durch zum Beispiel Schweiß oder Wasser ausgewaschen werden kann. Aufgrund der geringen Anbindungsfähigkeit des Farbstoffes an die jeweilige Matrix ist zudem die Ergiebigkeit des Farbstoffes im Färbeprozess gering und damit einhergehend kann die Intensität der Färbung von Matrices gering ausgeprägt sein. Bei Verwendung von, insbesondere synthetischen, Farbstoffen kann, insbesondere im humanen Anwendungsbereich, zudem eine geringe Verträglichkeit vorliegen.

Ascorbinsäurederivate als Farbstoffe, insbesondere von in 6- und/oder 5-Position durch Farbchromophore substituierte Ascorbinsäurederivate, sind beispielsweise aus WO 2012/028245 oder WO 2012/069476 bekannt. Bei der Herstellung und Lagerung dieser Verbindungen arbeitet man unter inerten Bedingungen, beispielsweise unter Sauerstoffausschluss, da die Substanzen durch den Ascorbinsäure-Grundkörper oxidationsempfindlich sind.

Somit besteht weiterhin ein Bedarf an, unter anderem, verträglichen und insbesondere hautverträglichen, Farbstoffen, die eine gute Adhäsion und/oder Anbindungsfähigkeit der Farbstoffmoleküle an die jeweilige Matrix aufweisen, so dass die jeweilige Matrix dauerhaft gefärbt werden kann.

Demzufolge beschäftigt sich die vorliegende Erfindung mit dem Problem, verbesserte oder zumindest alternative Farbstoffe zum Färben von Matrices anzugeben, die sich insbesondere durch eine verbessertes Färbeverhalten auszeichnen, deren Adhäsions- und/oder Anbindungsfähigkeit an die zum Färben vorgesehene Matrix verbessert ist und die eine positive Auswirkung auf die Feuchtigkeit der Matrix ausüben, was bei einer Anwendung auf Haare zu einer erhöhten Haarelastizität führt.

Erfindungsgemäß wird dieses Problem durch die Gegenstände der unabhängigen Ansprüche gelöst. Vorteilhafte Ausführungsformen sind Gegenstand der abhängigen Ansprüche.

Es wurde nun überraschend festgestellt, dass sich die Verbindungen der Formel I, wie nachfolgend beschrieben, in hervorragender Weise als protein-adhäsive Farbstoffe eignen. Unter dem Begriff protein-adhäsiver Farbstoff versteht man einen Farbstoff, der aufgrund mindestens einer Adhäsion an eine proteinhaltige Matrix bindet. Bevorzugte Matrices sind hierbei Haut, Haare und/oder Nägel oder mit anderen Worten keratinhaltige Substrate, insbesondere keratinhaltige Fasern. Bevorzugte keratinhaltige Fasern sind Haare. Besonders bevorzugte keratinhaltige Fasern sind menschliche Haare. Die den Zusammenhalt bewirkenden Kräfte für eine Adhäsion sind nicht vollständig erforscht, so dass es verschiedene Adhäsionstheorien gibt, die sowohl die mechanische Adhäsion aufgrund physikalisch-mechanischer Kräfte und die spezifische Adhäsion aufgrund chemischer und physikalischer Kräfte umfassen. Es ist beispielsweise möglich, dass die Verbindungen der Formel I, wie nachfolgend beschrieben, bedingt durch ihre Amphiphilie, mit den Matrixmolekülen, wie zuvor beschrieben, physikalisch wechselwirken oder eine kovalente Bindung an eine Amino- oder Thiolgruppe der Matrix ausbilden. Die proteinadhäsiven Farbstoffe können demzufolge mit der Matrix physikalisch wechselwirken oder eine kovalente Bindung ausbilden. Bedingt durch die Amphiphilie besitzen die Verbindungen der Formel I, wie nachfolgend beschrieben, auch oberflächenaktive Eigenschaften. Bevorzugt sind die Verbindungen der Formel I, wie nachfolgend beschrieben, haut- oder haaranhaftende und/oder haut-oder haarbindende Farbstoffe. Besonders bevorzugt sind die Verbindungen der Formel I, wie nachfolgend beschrieben, haaranhaftende und/oder haarbindende Farbstoffe.

Die Verbindungen der Formel I, wie nachfolgend beschrieben, sind ebenfalls filmbildende Farbstoffe. Auf Grund der homogenen Verteilung durch gleichmäßige Oberflächenadhäsion und/oder Bindung liegt ein wesentlicher Vorteil neben der langanhaltenden Haftung an der Matrix darin, dass der Farbstoff gleichmäßig auf der Matrix, insbesondere gleichmäßig auf der keratinhaltigen Faser verteilt wird und so zu einem einheitlichen Färbeergebnis über die gesamte Matrix beiträgt. Bei keratinhaltigen Fasern kann das einheitliche Färbeergebnis von der Wurzel bis zum Ende der Faser erzielt werden. Es ist für die vorliegende Erfindung unerheblich, ob die Matrix eine natürliche oder eine behandelte Matrix darstellt, so lange eine genügend große Anzahl an Amino- oder Thiolgruppen gewährleistet bleibt. Keratinhaltige Fasern können daher naturbelassen sein, oder auch künstlich verändert worden sein, beispielsweise in Form einer Dauerwelle, durch Blondierung oder Färbung mit konventionellen Haarfärbemitteln und Verfahren.

Erfindungsgemäße Verbindungen der Formel I, wie nachfolgend beschrieben, eignen sich als Antiglykierungsmittel (Antiglycation agents) und wirken der Bildung von AGEs (advanced glycation endproducts) entgegen.

Ein erster Gegenstand der Erfindung sind demzufolge die Verbindungen der Formel I, 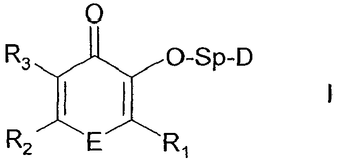 wobei
E NR₄ oder O bedeutet,
Sp eine Bindung, alk, -C(O)- oder -(CO)-alk bedeutet,
D ein Farbchromophor (wie in den Ansprüchen definiert) bedeutet,
R₁, R₂ oder R₃ jeweils unabhängig voneinander -H, -A, -OA, -(CH₂)ₚ-OH, -
C(O)OA, COOH oder COOX bedeuten,
p eine ganze Zahl von 1 bis 4 bedeutet,
X das Gegenion zu der [COO⁻]-Gruppe ist,
R₄ A bedeutet,
alk eine lineare oder verzweigte oder cyclische Alkylen-Gruppe mit 1 bis 18 C-Atomen bedeutet und
A eine lineare oder verzweigte Alkylgruppe mit 1 bis 20 C-Atomen bedeutet und/oder deren Salze, Tautomere, Stereoisomere und/oder Solvate, einschließlich deren Mischungen in allen Verhältnissen.

Ein weiterer Gegenstand der Erfindung ist demzufolge die Verwendung von Verbindungen der Formel I, 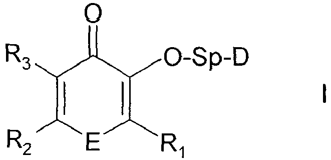 wobei
E NR₄ oder O bedeutet,
Sp eine Bindung, alk, -C(O)- oder -(CO)-alk bedeutet,
D ein Farbchromophor bedeutet,
R₁, R₂ oder R₃ jeweils unabhängig voneinander -H, -A, -OA, -(CH₂)ₚ-OH, -C(O)OA, COOH oder COOX bedeuten,
p eine ganze Zahl von 1 bis 4 bedeutet,
X das Gegenion zu der [COO⁻]-Gruppe ist,
R₄A bedeutet,
alk eine lineare oder verzweigte oder cyclische Alkylen-Gruppe mit 1 bis 18 C-Atomen bedeutet und
A eine lineare oder verzweigte Alkylgruppe mit 1 bis 20 C-Atomen bedeutet und/oder deren Salze, Tautomere, Stereoisomere und/oder Solvate, einschließlich deren Mischungen in allen Verhältnissen als protein-adhäsive Farbstoffe.

Im Rahmen der Erfindung sind die Verbindungen der Formel I, so definiert, dass man darunter auch pharmazeutisch oder kosmetisch verwendbare Derivate, Salze, Hydrate, Solvate, Vorstufen der Verbindungen, Tautomere und optisch aktive Formen (wie z.B. Stereoisomere, Diastereomere, Enantiomere, Racemate) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch oder kosmetisch verwendbaren Derivaten versteht man z.B. die Salze der erfindungsgemäßen Verbindungen. Die Verbindungen der Formel I können cis-trans-Isomere oder Tautomere bilden. Die Formel I schließt alle diese Formen ein.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern.

Andererseits können Säuren der Formel I mit Basen (z.B. Natrium- oder Kaliumhydroxid oder -carbonat) in die entsprechenden Metall-, insbesondere Alkalimetall- oder Erdalkalimetall- oder in die entsprechenden Ammoniumsalze umgewandelt werden.

Die Abkürzung "A" steht für Alkyl mit 1 bis 20 C-Atomen, d.h. mit anderen Worten für eine lineare oder verzweigte Alkylgruppe mit 1 bis 20 C-Atomen, beispielsweise Methyl, Ethyl, Isopropyl, Propyl, Butyl, sec-Butyl oder tert-Butyl, weiterhin Pentyl, 1-, 2- oder 3-Methylbutyl, 1,1-, 1,2- oder 2,2-Dimethylpropyl, 1-Ethylpropyl, n-Hexyl, n-Heptyl, n-Octyl, weiterhin Nonyl, Decyl, Undecyl, Dodecyl, Tridecyl, Tetradecyl, Pentadecyl, Hexadecyl, Heptadecyl, Oktadecyl, Nonadecyl und Eicosyl. Bevorzugt wird aus der geradkettigen oder verzweigten Alkylgruppe mit 1 bis 20 C-Atomen die geradkettige oder verzweigte Alkylgruppe mit 1 bis 8 C-Atomen, d.h. Methyl, Ethyl, Isopropyl, Propyl, Butyl, sec-Butyl oder tert-Butyl, weiterhin Pentyl, 1-, 2- oder 3-Methylbutyl, 1,1-, 1,2- oder 2,2-Dimethylpropyl, 1-Ethylpropyl, n-Hexyl, n-Heptyl, n-Octyl, ausgewählt. Besonders bevorzugt wird aus der geradkettigen oder verzweigten Alkylgruppe mit 1 bis 20 C-Atomen die geradkettige oder verzweigte Alkylgruppe mit 1 bis 4 C-Atomen ausgewählt, d.h. Methyl, Ethyl, Isopropyl, Propyl, Butyl, sec-Butyl oder tert-Butyl.

Die Abkürzung "alk" steht für eine lineare oder verzweigte oder cyclische Alkylen-Gruppe mit 1 bis 18 C-Atomen wird von den zuvor beschriebenen Alkylgruppen mit 1 bis 18 C-Atomen abgeleitet. Bevorzugt steht alk für Methylen, Ethylen, Propylen oder Butylen.

Die Abkürzung -OA steht für O-Alkyl mit 1 bis 20 C-Atomen, gleichbedeutend für Alkoxy mit 1 bis 20 C-Atomen, wobei A wie zuvor definiert verwendet wird. Entsprechend steht die Abkürzung -NHA für Alkylamino und -NA₂ für Dialkylamino.

X beschreibt das Gegenion für die Kationen [NHA₂]⁺ und [NA₃]⁺, wobei A eine der zuvor angegebenen Bedeutungen hat, vorzugsweise Cl⁻, Br⁻, I⁻oder [SO₄]²⁻ oder das Gegenion des Anions [COO]⁻ oder [SO₃]⁻, vorzugsweise ein Ammoniumion oder ein Alkalimetall- oder Erdalkalimetallkation wie Na⁺, K⁺, Mg²⁺ oder Ca²⁺.

In einer Ausführungsform sind Verbindungen der Formel I bevorzugt, bei denen der Substituent R₁ A bedeutet und A eine der zuvor angegebenen Bedeutungen oder als bevorzugt angegebenen Bedeutungen hat. Besonders bevorzugt ist R₁ Methyl oder Ethyl. Ganz besonders bevorzugt ist R₁ Methyl.

In einer Ausführungsform sind Verbindungen der Formel I bevorzugt, bei denen der Substituent R₂ H -(CH₂)ₚ-OH, -C(O)OA, COOH oder COOX bedeutet, wobei A, X und p eine zuvor angegebene oder bevorzugt angegebene Bedeutung haben. Besonders bevorzugt ist der Substituent R₂ H, Hydroxymethyl oder -C(O)OEthyl oder-C(O)OMethyl. Ganz besonders bevorzugt ist der Substituent R₂ H.

In einer Ausführungsform sind Verbindungen der Formel I bevorzugt, bei denen der Substituent R₃ H bedeutet.

In einer bevorzugten Ausführungsform sind Verbindungen der Formel I bevorzugt, bei denen die Substituenten R₂ und R₃ H bedeuten.

In einer Ausführungsform sind Verbindungen der Formel I bevorzugt, bei denen der Substituent R₄ A bedeutet und A eine der als bevorzugt angegebenen Bedeutungen hat. Besonders bevorzugt ist R₄ Methyl oder Ethyl. Ganz besonders bevorzugt ist R₄ Methyl.

In einer bevorzugten Ausführungsform sind Verbindungen der Formel I bevorzugt, bei denen die Variable E O oder NR₄ bedeutet und die Substituenten R₄, R₃, R₂ und R₁ eine der zuvor angegebenen oder nachfolgend angegebenen bevorzugten Bedeutungen oder besonders bevorzugt angegebenen Bedeutungen haben.

Demzufolge sind die Verbindungen der Formeln I-a, I-b, I-c und I-d besonders bevorzugt: 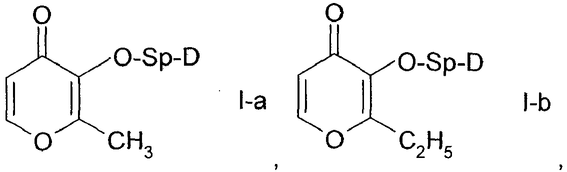 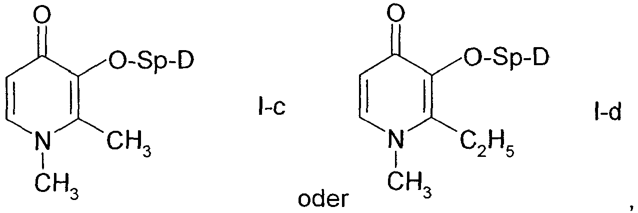 wobei Sp und D eine der zuvor oder nachfolgend angegebenen Bedeutungen haben oder eine der nachfolgend angegebenen bevorzugten Bedeutungen haben.

In einer Ausführungsform sind Verbindungen der Formel I bevorzugt, bei denen die Variable E O bedeutet.

In einer Ausführungsform sind Verbindungen der Formel I bevorzugt, bei denen die Variable E N-R₄ bedeutet und R₄ A bedeutet, wobei A eine der zuvor angegebenen oder bevorzugt angegebenen Bedeutungen hat. Bevorzugt bedeutet die Variable E N-Methyl.

In einer bevorzugten Ausführungsform sind Verbindungen der Formel I bevorzugt, bei denen die Variable E O bedeutet und die Substituenten R₃, R₂, R₁, Sp und D eine der zuvor angegebenen oder nachfolgend angegebenen bevorzugten Bedeutungen oder besonders bevorzugt angegebenen Bedeutungen haben. In dieser Ausführungsform sind die Verbindungen der Formel I-a oder I-b besonders bevorzugt. In dieser Ausführungsform sind die Verbindungen der Formel I-a ganz besonders bevorzugt.

In einer bevorzugten Ausführungsform sind Verbindungen der Formel I bevorzugt, bei denen die Variable E NR₄ bedeutet und die Substituenten R₄, R₃, R₂, R₁, Sp und D eine der zuvor angegebenen oder nachfolgend angegebenen bevorzugten Bedeutungen oder besonders bevorzugt angegebenen Bedeutungen haben. In dieser Ausführungsform sind die Verbindungen der Formel I-c oder I-d besonders bevorzugt. In dieser Ausführungsform sind die Verbindungen der Formel I-c ganz besonders bevorzugt.

In einer bevorzugten Ausführungsform sind Verbindungen der Formel I, I-a, I-b, I-c oder I-d bevorzugt, bei denen die Variable Sp -C(O)- bedeutet.

Die vorteilhaften Eigenschaften der Verbindungen der Formel I, I-a, I-b, I-c oder I-d, wie zuvor beschrieben, werden durch die Struktureinheit der Formel I-1 erzielt, 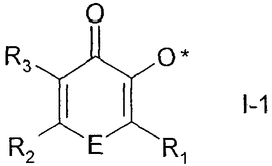 wobei R₁, R₂, R₃ und E eine zuvor für die Verbindungen der Formel I, I-a, I-b, I-c und I-d genannte Bedeutung haben oder eine als bevorzugt angegebene Bedeutung haben.

Das Farbchromophor D leitet sich von einem Farbstoff ab, der die Matrix, wie zuvor beschrieben, färben kann und/oder welcher selbst gefärbt ist, d.h. in der Lage ist, Strahlung in dem Bereich zwischen 250 nm und 800 nm zu absorbieren. Bevorzugt leitet sich das Farbchromophor D von einem Farbstoff ab, der Strahlung im sichtbaren Bereich zwischen 400 und 700 nm absorbiert.

Das Farbchromophor D kann auch von einem fluoreszierenden Farbstoff abgeleitet sein, so dass eine Verbindung der Formel I, I-a, I-b, I-c oder I-d enthaltend dieses fluoreszierende Farbchromophor auch auf einer bereits dunkel erscheinenden Matrix sichtbar wird.

Ein fluoreszierendes Farbchromophor D leitet sich von einem fluoreszierenden Farbstoff ab, der die Matrix, wie zuvor beschrieben, färben kann und/oder welcher selbst gefärbt ist, d.h. in der Lage ist, Strahlung in dem Bereich zwischen 250 nm und 800 nm zu absorbieren und welcher mindestens einen Teil des absorbierten Lichts wieder emittieren kann. Bevorzugt leitet sich das fluoreszierende Farbchromophor D von einem fluoreszierenden Farbstoff ab, der Strahlung im sichtbaren Bereich zwischen 400 und 800 nm absorbiert und Strahlung zwischen 410 und 810 nm emittiert. Besonders bevorzugt leitet sich das fluoreszierende Farbchromophor D von einem fluoreszierenden Farbstoff ab, der Strahlung im sichtbaren Bereich zwischen 420 und 550 nm absorbiert und Strahlung zwischen 470 und 600 nm emittiert.

Das Farbchromophor D kann demzufolge von den folgenden Farbstoffen abgeleitet werden: (solange es strukturell vom Anspruchsumfang umfasst ist) Acridinfarbstoffe, Acridonfarbstoffe, Anthrapyrimidinfarbstoffe, Anthrachinonfarbstoffe, Azinfarbstoffe, (Poly)-Azofarbstoffe, Hydrazonofarbstoffe, Hydrazonfarbstoffe, bevorzugt Arylhydrazonfarbstoffe, Azomethinfarbstoffe, Benzanthronfarbstoffe, Benzimidazolfarbstoffe, Benzimidazolonfarbstoffe, Benzindolfarbstoffe, Benzoxazolfarbstoffe, Benzopyranfarbstoffe, Benzothiazolfarbstoffe, Benzochinonfarbstoffe, Bisazinfarbstoffe, Bis-Isoindolinfarbstoffe, Carboxanilidfarbstoffe, Coumarinfarbstoffe, Cyaninfarbstoffe, beispielsweise Merocyaninfarbstoffe, Azacarbocyaninfarbstoffe, Diazacarbocyaninfarbstoffe, Diazahemicyaninfarbstoffe, Hemicyaninfarbstoffe oder Tetraazacarbocyaninfarbstoffe, Diazinfarbstoffe, Diketopyrrolopyrrolfarbstoffe, Dioxazinfarbstoffe, Diphenylaminfarbstoffe, Diphenylmethanfarbstoffe, Dithiazinfarbstoffe, Flavonoide beispielsweise Flavanthrone und Flavone, Fluorindinfarbstoffe, Formazanfarbstoffe, Indaminfarbstoffe, Indanthronfarbstoffe, Indigoide und Pseudo-Indigoide, Indophenolfarbstoffe, Indoanilinfarbstoffe, Isoindolinfarbstoffe, Isoindolinonfarbstoffe, Isoviolanthronfarbstoffe, Lactonfarbstoffe, (Poly)methinfarbstoffe, beispielsweise Dimethinfarbstoffe vom Stilben- oder Styrol-Typ, Naphthalimidfarbstoffe, Naphthanilidfarbstoffe, Naphtholactamfarbstoffe, Naphthochinonfarbstoffe, Farbstoffe enthaltend mindestens eine NO₂-Gruppe, Oxadiazolfarbstoffe, Oxazinfarbstoffe, Perilonfarbstoffe, Perinonfarbstoffe, Perylenfarbstoffe, Phenazinfarbstoffe, Phenoxazinfarbstoffe, Phenothiazinfarbstoffe, Phthalocyaninfarbstoffe, Polyene oder Carotinoide, Porphyrinfarbstoffe, Pyranthronfarbstoffe, Pyrazolanthronfarbstoffe, Pyrazolonfarbstoffe, Pyrimidinoanthronfarbstoffe, Pyroninfarbstoffe, Chinacridonfarbstoffe, Chinolinfarbstoffe, Chinophathalonfarbstoffe, Swuaranfarbstoffe, Tetrazolfarbstoffe, Thiazinfarbstoffe, Thioindigofarbstoffe, Thiopyroninfarbstoffe, Triarylmethanfarbstoffe oder Xanthenfarbstoffe.

Erfindungsgemäß sind Verbindungen der Formel I, I-a, I-b, I-c oder I-d, wie zuvor beschrieben oder als bevorzugt beschrieben, bevorzugt, wenn das Farbchromophor D einer der Formeln II, III, IV, V, VI, VII, VIII, IX, X, XI oder XII entspricht, wobei die Markierung * in den im folgenden beschriebenen Formeln die Anknüpfungsstelle an den Spacer Sp bedeutet, bevorzugt die Anknüpfungsstelle an den Spacer -C(O)- bedeutet: 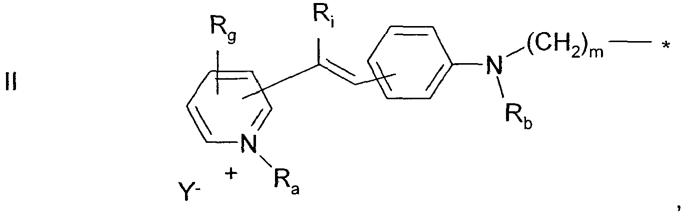 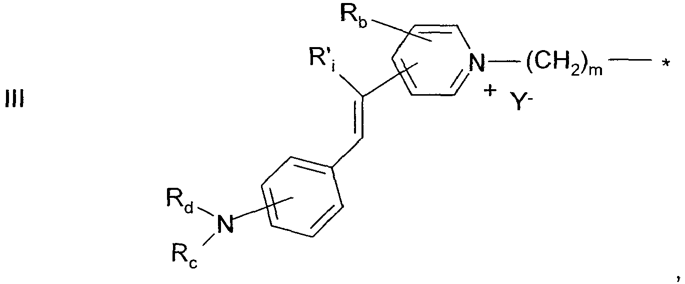 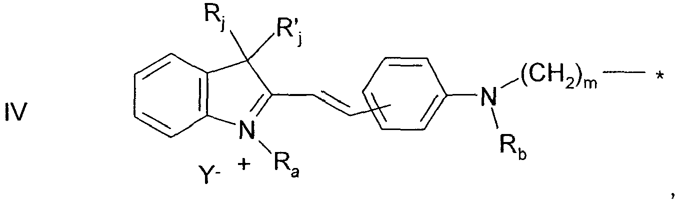 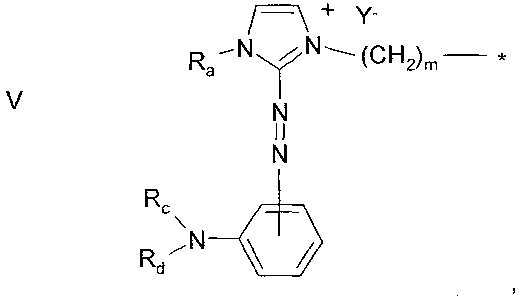 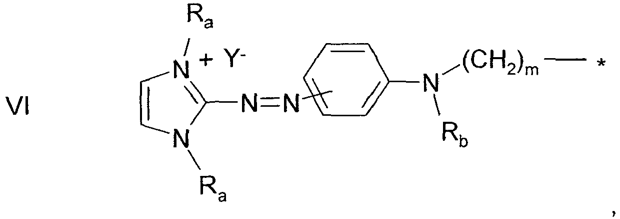 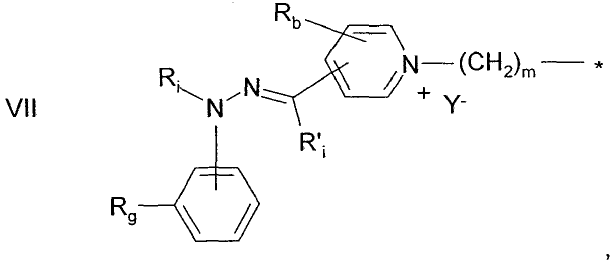 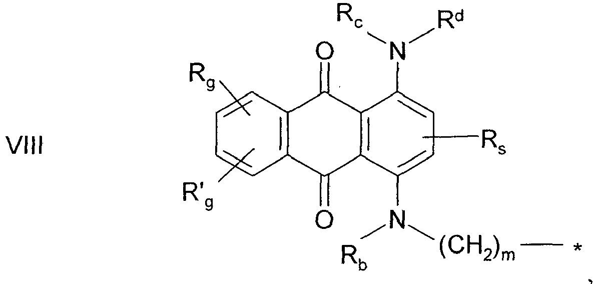 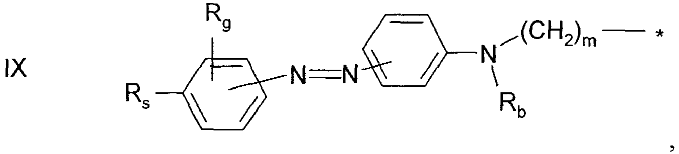 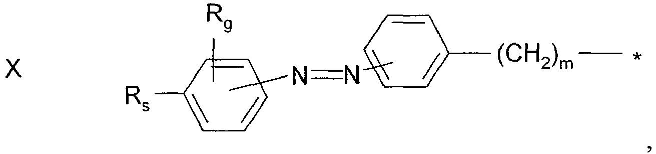 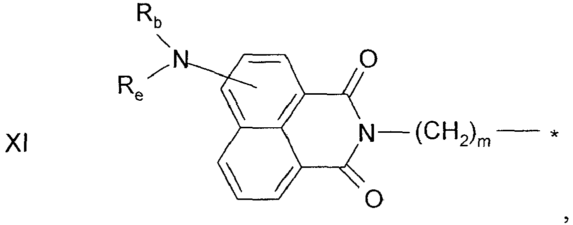 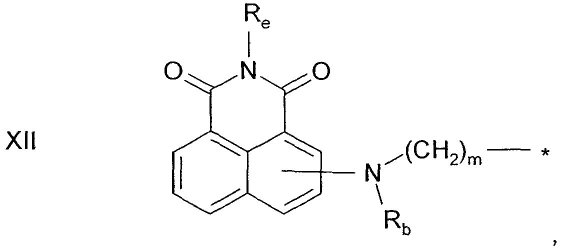 wobei
Rₐ, Rⱼ und R'ⱼ jeweils unabhängig voneinander A bedeuten,
R_{b} H oder A bedeutet,
R_{g} und R'g jeweils unabhängig voneinander H, Hal, NA₂, CN, COOH, OH, CF₃, OA, OC(O)A, C(O)OA, NHC(O)A, NHSO₂A, SO₂NA₂ bedeuten,
Rₛ H, A, NA₂, OA oder SO₃Y bedeutet,
Rᵢ und R'ᵢ jeweils unabhängig voneinander H oder A bedeuten,
R_{c} und R_{d} jeweils unabhängig voneinader H oder A bedeuten, wobei A mit mindestens einer OH-Gruppe substituiert sein kann,
Rₑ eine Alkylgruppe mit 1 bis 6 C-Atomen bedeutet, die durch mindestens eine Gruppe NA₂ oder NA₃Y substituiert ist,
Y ein Anion einer organischen oder anorganischen Säure oder ein Kation ist,
A eine lineare oder verzweigte Alkylgruppe mit 1 bis 20 C-Atomen bedeutet,
Hal F, Cl, Br oder I bedeutet und
m 0, 1, 2, 3, 4 oder 5 bedeutet.

Die Abkürzung "A" wurde zuvor beschrieben und die Ausführungen zu "A", "OA", "NHA", NA₂" gelten auch für die Formeln II bis XII entsprechend.

Hal bedeutet Halogen oder mit anderen Worten F, Cl, Br oder I. Hal ist bevorzugt Br oder I.

Das Anion Y entspricht einer organischen oder anorganischen Säure, die pharmakologisch oder aus kosmetischer Sicht verträglich ist oder einem Kation. Beispiele für das Anion Y sind wie zuvor beschrieben F⁻, Cl⁻, Br⁻, I⁻oder [SO₄]²⁻. Beispiele für Y als Kation umfassen Na⁺, K⁺, Mg⁺⁺, Ca⁺⁺, NH₄⁺ und quaternäre Ammoniumsalze.

Die Variable m bedeutet 0, 1, 2, 3, 4 oder 5. Bevorzugte Bedeutungen werden bei den Formeln II bis XII spezifisch angegeben.

Im Folgenden werden bevorzugte Ausführungsformen für das Farbchromophor D gelistet, der durch die Formeln II, III, IV, V, VI, VII, VIII, IX, X, XI und XII beschrieben wird. Jede bevorzugte oder besonders bevorzugte Ausführungsform einer der genannten Formeln II bis XII kann mit einer oder mehreren der genannten bevorzugten oder besonders bevorzugten Ausführungsformen der übrigen Formeln II bis XII kombiniert werden, um die Auswahl des Farbchromophors D in den Verbindungen der Formel I, I-a, I-b, I-c oder I-d bevorzugt zu beschreiben.

In einer Ausführungsform für die Substituenten der Formel II ist Rᵢ H. In einer weiteren Ausführungsform für die Substituenten der Formel II ist Rᵢ H und Rₐ und R_{b} sind jeweils unabhängig voneinander A, wobei A eine der zuvor angeführten Bedeutungen oder als bevorzugt angegebenen Bedeutungen hat. In einer weiteren Ausführungsform für die Substituenten der Formel II ist Rᵢ H und Rₐ und R_{b} sind jeweils unabhängig voneinander A, wobei A eine der zuvor angeführten Bedeutungen oder als bevorzugt angegebenen Bedeutungen hat und R_{g} ist H. In Formel II ist m bevorzugt 1. In Formel II ist Y bevorzugt Chlorid, Bromid, ein Alkylsulfat oder Alkylethersulfat, wie z.B. Laurylsulfat oder Laurylethersulfat.

In einer Ausführungsform für die Substituenten der Formel III ist R'ᵢ H. In einer weiteren Ausführungsform für die Substituenten der Formel III ist R'ᵢ H und R_{c} und R_{d} sind jeweils unabhängig voneinander A, wobei A mit mindestens einer OH-Gruppe substituiert sein kann. A hat dabei eine der zuvor angeführten Bedeutungen oder als bevorzugt angegebenen Bedeutungen, wobei die genannten Alkylgruppen mit 1 bis 20 C-Atomen zusätzlich mit mindestens einer OH-Gruppe substituiert sein können. In einer weiteren Ausführungsform für die Substituenten der Formel III ist R'ᵢ H und R_{c} und R_{d} sind jeweils gleich, wobei A bevorzugt ausgewählt wird aus einer linearen oder verzweigten Alkylgruppe mit 1 bis 8 C-Atomen oder einer linearen oder verzweigten Alkylgruppe mit 1 bis 8 C-Atomen, die mit einer OH-Gruppe substituiert ist. In Formel III werden R_{c} und R_{d} besonders bevorzugt ausgewählt aus Methyl oder 2-Hydroxy-ethyl. In Formel III ist R_{b} bevorzugt H. In Formel III ist m bevorzugt 2 oder 3, besonders bevorzugt 2. In Formel III ist Y bevorzugt Chlorid, Bromid, ein Alkylsulfat oder Alkylethersulfat, wie z.B. Laurylsulfat oder Laurylethersulfat.

In einer bevorzugten Ausführungsform der Substituenten der Formel III ist R'ᵢ H, R_{c} und R_{d} sind gleich und werden ausgewählt aus einer linearen oder verzweigten Alkylgruppe mit 1 bis 8 C-Atomen oder einer linearen oder verzweigten Alklylgruppe mit 1 bis 8 C-Atomen, die mit einer OH-Gruppe substituiert ist, R_{g} ist H und m ist 3 oder 4.

In einer Ausführungsform für die Substituenten der Formel IV sind Rⱼ und R'ⱼ Methyl. In einer weiteren Ausführungsform für die Substituenten der Formel IV sind Rⱼ und R'ⱼ Methyl und Rₐ und R_{b} sind jeweils unabhängig voneinander A, wobei A eine der zuvor angeführten Bedeutungen oder als bevorzugt angegebenen Bedeutungen hat. In Formel IV ist m bevorzugt 1. In Formel IV ist Y bevorzugt Chlorid, Bromid, ein Alkylsulfat oder Alkylethersulfat, wie z.B. Laurylsulfat oder Laurylethersulfat.

In einer Ausführungsform für die Substituenten der Formel V ist Rₐ Methyl. In einer weiteren Ausführungsform für die Substituenten der Formel V ist Rₐ Methyl und R_{c} und R_{d} sind jeweils gleich, wobei A bevorzugt ausgewählt wird aus einer linearen oder verzweigten Alkylgruppe mit 1 bis 8 C-Atomen oder einer linearen oder verzweigten Alkylgruppe mit 1 bis 8 C-Atomen, die mit einer OH-Gruppe substituiert ist. In Formel V werden R_{c} und R_{d} besonders bevorzugt ausgewählt aus Methyl oder 2-Hydroxy-ethyl. In Formel V ist m bevorzugt 3. In Formel V ist Y bevorzugt Chlorid, Bromid, ein Alkylsulfat oder Alkylethersulfat, wie z.B. Laurylsulfat oder Laurylethersulfat.

In einer Ausführungsform für die Substituenten der Formel VI ist R_{b} Methyl. In einer weiteren Ausführungsform für die Substituenten der Formel VI ist R_{b} Methyl und Rₐ ist jeweils identisch, wobei A eine der zuvor angeführten Bedeutungen oder als bevorzugt angegebenen Bedeutungen hat. In Formel VI ist m bevorzugt 1. In Formel VI ist Y bevorzugt Chlorid, Bromid, ein Alkylsulfat oder Alkylethersulfat, wie z.B. Laurylsulfat oder Laurylethersulfat.

In einer Ausführungsform für die Substituenten der Formel VII sind Rᵢ und R'ᵢ H. In einer weiteren Ausführungsform für die Substituenten der Formel VII sind Rᵢ und R'ᵢ H und R_{b} ist H. In Formel VII ist R_{g} bevorzugt H. In Formel VII ist m bevorzugt 3 oder 4, besonders bevorzugt 3. In Formel VII ist Y bevorzugt Chlorid, Bromid, ein Alkylsulfat oder Alkylethersulfat, wie z.B. Laurylsulfat oder Laurylethersulfat.

In einer Ausführungsform für die Substituenten der Formel VIII ist R_{b} H oder Methyl. In einer weiteren Ausführungsform für die Substituenten der Formel VIII ist R_{b} H oder Methyl und R_{g} und R'_{g} sind H. In Formel VIII sind R_{c} und R_{d} bevorzugt beide H oder beide Methyl. In Formel VIII ist m bevorzugt 3 oder 4, besonders bevorzugt 3. In Formel VIII ist Y bevorzugt Natrium, Kalium, Ammonium oder eine quaternäre Ammoniumverbindung.

In einer Ausführungsform für die Substituenten der Formel IX ist R_{b} Methyl. In einer weiteren Ausführungsform für die Substituenten der Formel IX ist R_{b} Methyl und R_{g} ist H. In Formel IX ist m bevorzugt 1. In Formel IX ist Y Natrium, Kalium, Ammonium oder eine quaternäre Ammoniumverbindung.

In einer Ausführungsform für die Substituenten der Formel X ist m 0. In einer weiteren Ausführungsform für die Substituenten der Formel X ist m 0 und R_{g} ist H. In Formel X ist Rₛ bevorzugt H oder NA₂, wobei A eine zuvor angegebene Bedeutung oder eine als bevorzugt angegebene Bedeutung hat.

In einer Ausführungsform für die Substituenten der Formel XI ist R_{b} H. In einer weiteren Ausführungsform für die Substituenten der Formel XI ist R_{b} H und Rₑ ist eine Alkylgruppe mit 3 C-Atomen, die durch mindestens eine Gruppe NA₂ oder NA₃Y substituiert ist, wobei A eine zuvor angegebene Bedeutung oder eine als bevorzugt angegebene Bedeutung hat. In Formel XI ist m bevorzugt 5. In Formel XI ist Y bevorzugt Chlorid, Bromid, ein Alkylsulfat oder Alkylethersulfat, wie z.B. Laurylsulfat oder Laurylethersulfat.

In einer Ausführungsform für die Substituenten der Formel XII ist R_{b} Methyl. In einer weiteren Ausführungsform für die Substituenten der Formel XII ist R_{b} Methyl und Rₑ ist eine Alkylgruppe mit 4 C-Atomen, die durch mindestens eine Gruppe NA₂ oder NA₃Y substituiert ist, wobei A eine zuvor angegebene Bedeutung oder eine als bevorzugt angegebene Bedeutung hat. In Formel XII ist m bevorzugt 1. In Formel XII ist m bevorzugt 5. In Formel XI ist Y bevorzugt Chlorid, Bromid, ein Alkylsulfat oder Alkylethersulfat, wie z.B. Laurylsulfat oder Laurylethersulfat.

Besonders bevorzugte Ausführungsformen des Restes D sind in den folgenden Teilstrukturen zu sehen:

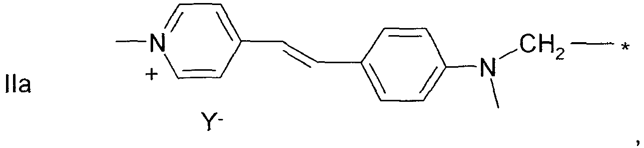

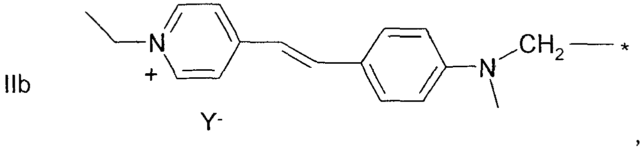

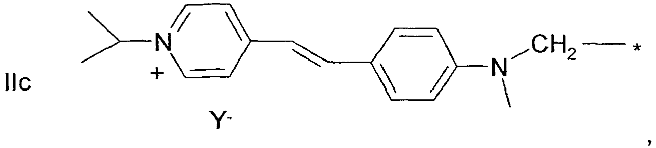

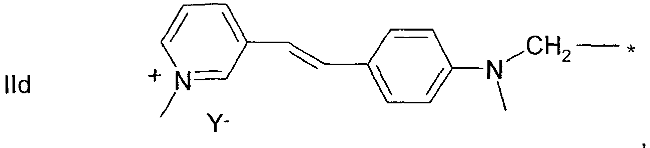

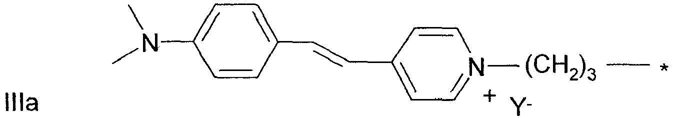

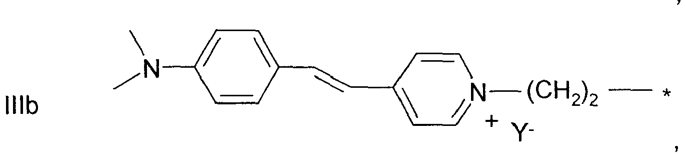

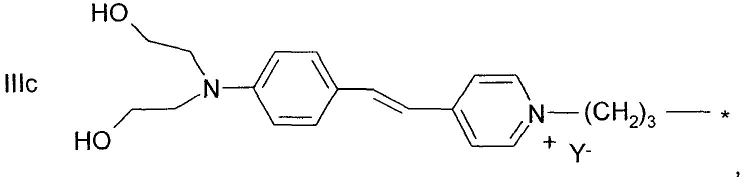

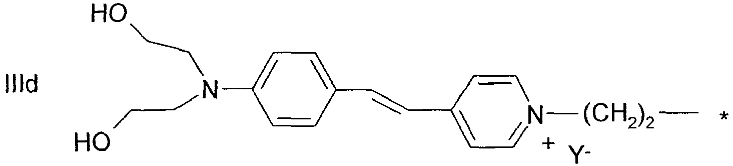

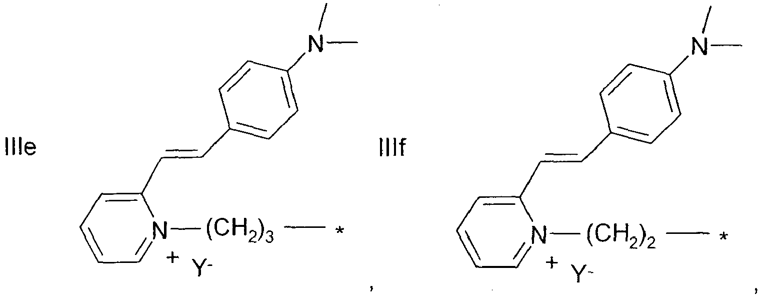

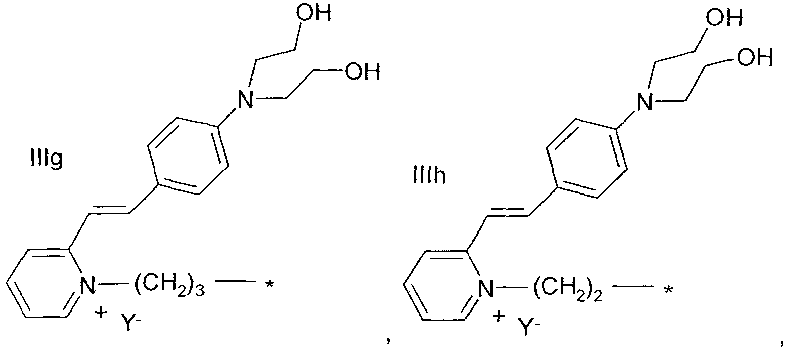

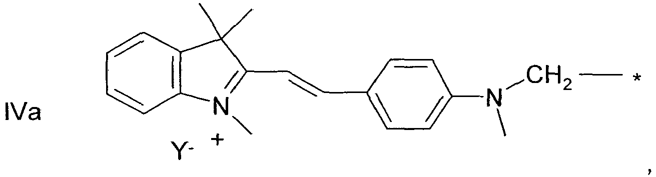

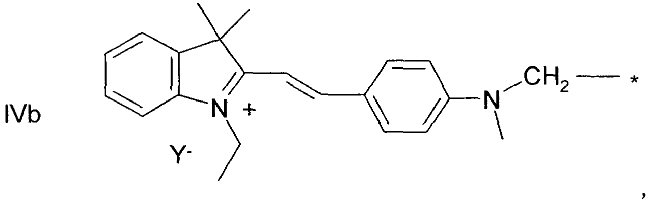

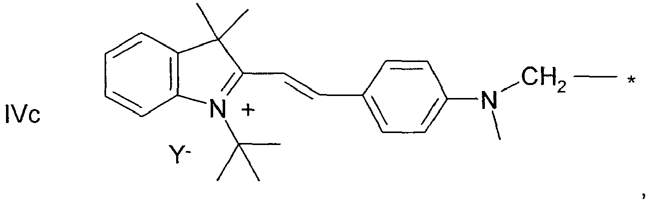

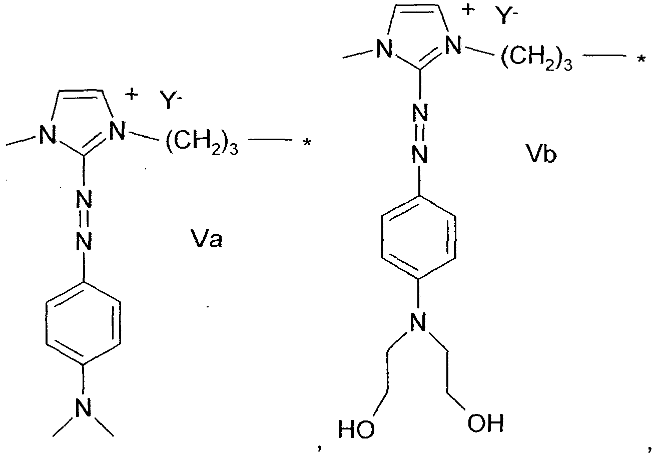

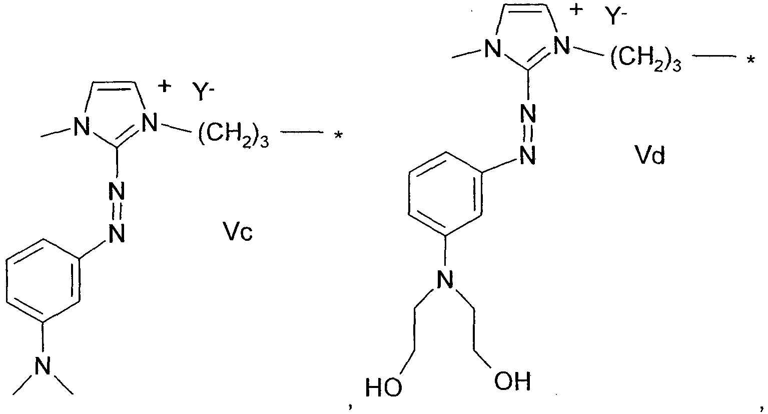

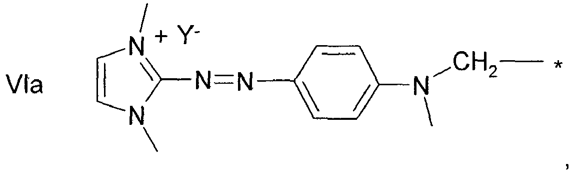

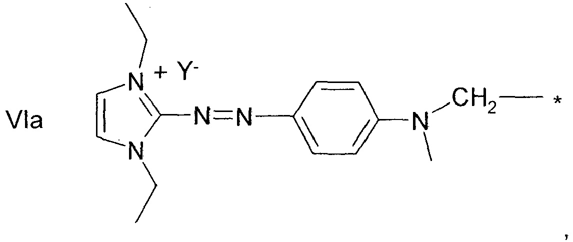

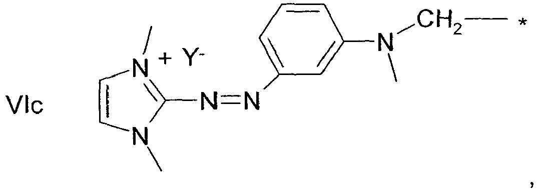

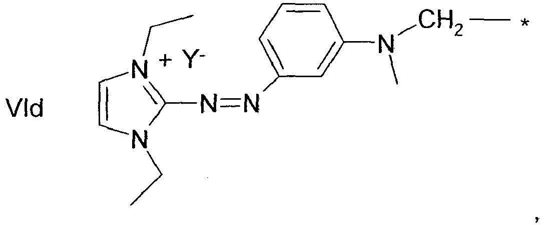

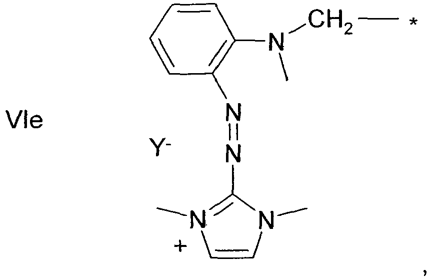

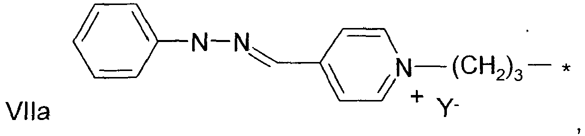

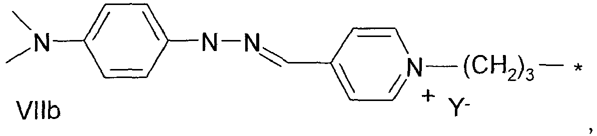

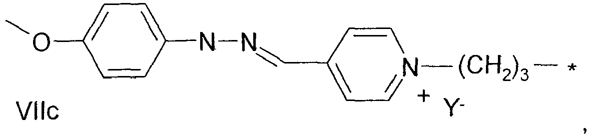

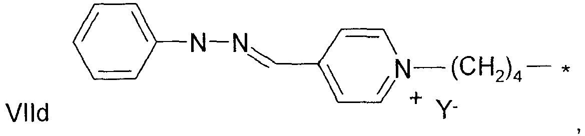

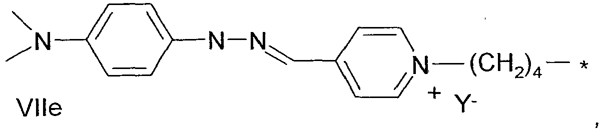

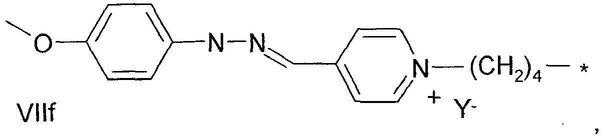

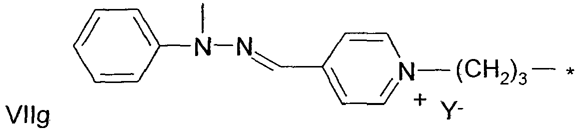

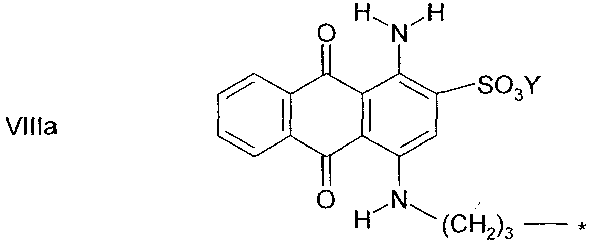

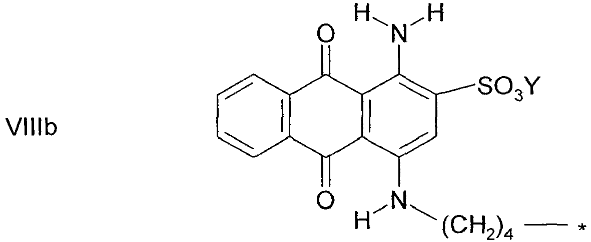

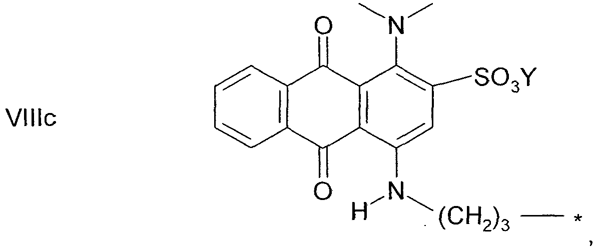

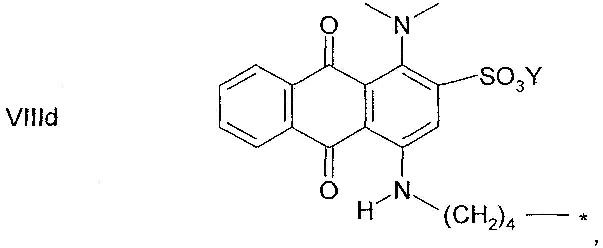

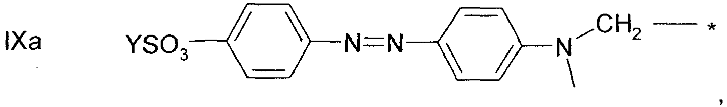

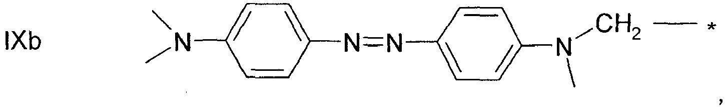

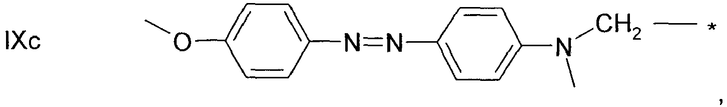

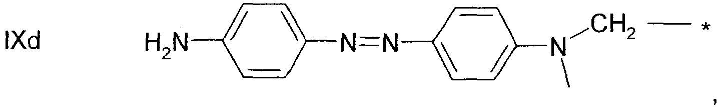

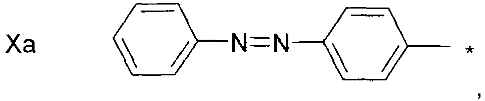

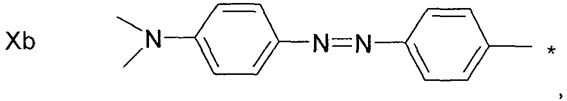

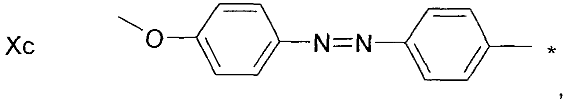

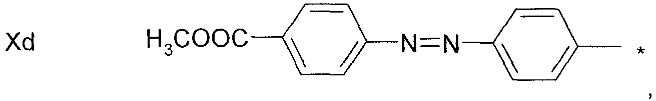

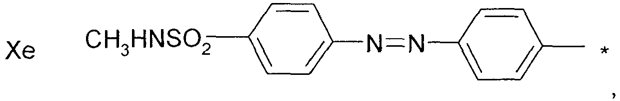

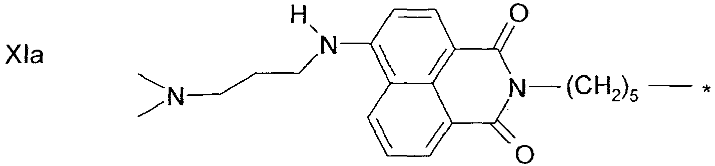

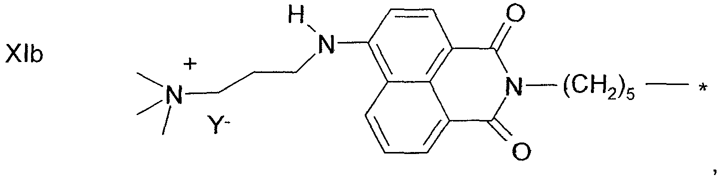

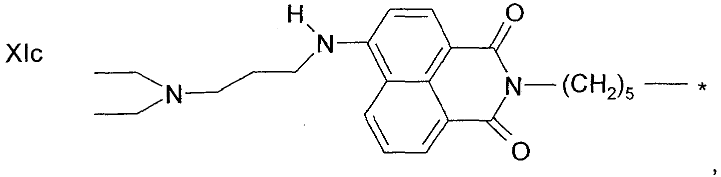

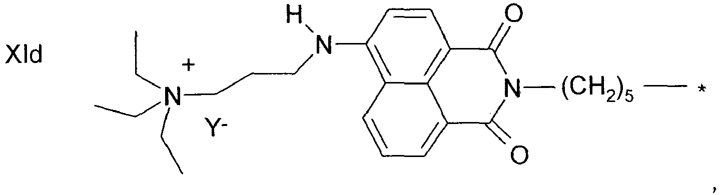

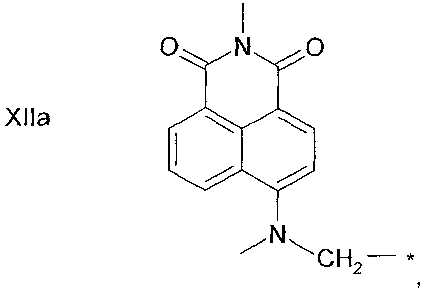

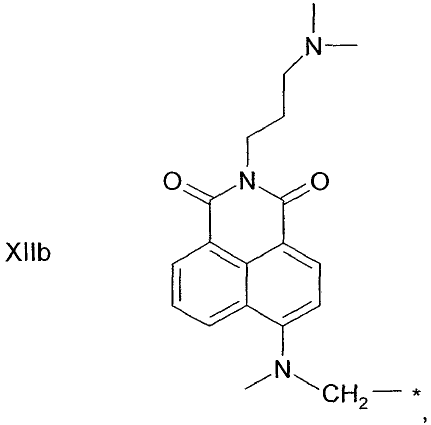

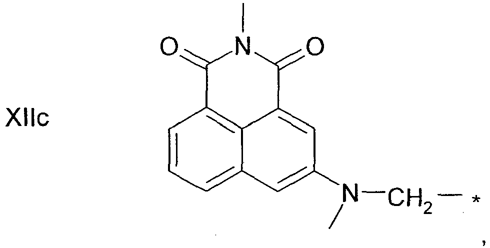

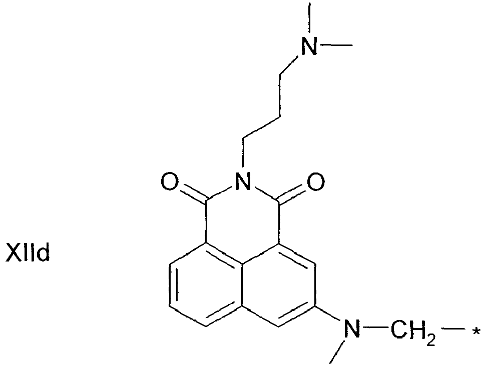

Bevorzugte Farbchromophore D entsprechen den Formeln III, X oder XI, wobei die Reste R'ᵢ, R_{b}, R_{c}, R_{d}, m, Y, Rₛ, R_{g} und Rₑ eine der zuvor oder bevorzugt angegebenen Bedeutungen haben oder den entsprechenden Teilformeln IIIa-IIIg, Xa-Xe oder XIa-XId entsprechen.

Ganz besonders bevorzugt entspricht D der Formel X, wobei R_{g} und Rₛ eine zuvor angegebene Bedeutung oder bevorzugte Bedeutung haben, insbesondere den Teilformeln Xa bis Xe entsprechen.

Die Herstellung der Verbindungen der Formel I, I-a, I-b, I-c, I-d, wie zuvor beschrieben, kann dabei nach dem Fachmann an sich aus der Literatur bekannten Methoden erfolgen. Die Reaktionsbedingungen für Veresterungen oder Veretherungen sind gängiger Stand der Technik und das Auswählen der geeigneten Reaktionsbedingungen gehört zum Standardfachwissen des Synthesefachmanns.

Gegenstand der Erfindung ist ebenfalls ein Verfahren zur Herstellung von Verbindungen der Formel I, I-a, I-b, I-c, I-d, wie zuvor beschrieben oder als bevorzugt beschrieben, dadurch gekennzeichnet, dass eine Verbindung der Formel XIII 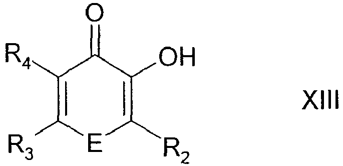 worin R₂, R₃, R₄ und E eine zuvor genannte Bedeutung haben, mit einer Verbindung der Formel XIV

D-Sp-M XIV

umgesetzt wird,
worin D und Sp eine zuvor genannte Bedeutung haben, und M Alkalimetall- oder Erdalkalimetallkation, Halogen, OH oder H bedeutet
oder
eine Verbindung der Formel XIII, worin R₂, R₃, R₄ und E zuvor genannte Bedeutung haben,
mit einem Aktivester der Verbindung der Formel XIV umgesetzt wird, abgeleitet von der freien Säure der Formel XIV, in der M OH und Sp -C(O)-bedeutet und D eine zuvor genannte Bedeutung hat.

Die Verbindungen der Formel XIII sind zum Teil kommerziell erhältlich, beispielsweise 3-Hydroxy-2-methyl-4-pyranon oder können nach bekannten Literaturmethoden hergestellt werden, beispielweise basierend auf R. Suzuki et al, Heterocyles, 1977, 6(9-10), 1575-80 oder A. Fassihi et al, European Journal of Medicinal Chemistry, 2009, 44(5), 2145-2157).

Die Verbindungen der Formel XIV sind zum Teil kommerziell erhältlich oder können nach Methoden synthetisiert werden, die z.B. in den Standardwerken wie Houben-Weyl, Methoden der organischen Chemie, Georg-Thieme-Verlag, Stuttgart beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Kommerziell erhältliche Farbstoffe sind z.B. gelistet im Colour Index International.

Die Etherbildung durch Umsetzung von Verbindungen der Formel XIV, in denen Sp eine Einfachbindung oder alk bedeutet und bei denen M = Hal bedeutet mit einer Verbindung der Formel XIII, wie zuvor beschrieben, findet vorzugsweise in Gegenwart von Triphenylphosphin und Diisopropylazodicarboxylat, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30° statt. Die Reaktionszeit liegt je nach den angewendeten Bedingungen zwischen einigen Minuten und mehreren Tagen.

Werden für die Synthese der Verbindungen der Formel I, wie zuvor beschrieben ein Säurehalogenid oder ein Aktivester der freien Säure der Formel XIV eingesetzt, so findet eine klassische nukleophile Substitution statt. Die Reaktionsbedingungen einer nukleophilen Substitution sind dem Synthesefachmann hinlänglich bekannt.

Bevorzugte Säurehalogenide der Formel XIV sind Säurechloride.

Wird eine Verbindung der Formel XIII, wie zuvor beschrieben, beispielsweise 3-Hydroxy-2-methyl-4-pyranon, mit einer Verbindung der Formel XIV umgesetzt, wobei Sp -C(O)- ist und M = H ist und D ein Farbchromophor bedeutet, wie zuvor beschrieben, so findet die Kupplungsreaktion vorzugsweise in Gegenwart eines Dehydratisierungsmittels, z.B. eines Carbodiimids wie Dicyclohexylcarbodiimid (DCC), N-(3-Dimethylaminopropyl)-N'-ethyl-carbodiimid-hydrochlorid (EDC) oder Diisopropylcarbodiimid (DIC), ferner z.B. Propanphosphonsäureanhydrid (vgl. Angew. Chem. 1980, 92, 129), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30° statt. Die Reaktionszeit liegt je nach den angewendeten Bedingungen zwischen einigen Minuten und mehreren Tagen.

Anstelle von Verbindungen der Formel XIV, wie zuvor definiert, können auch Derivate der Formel XIV, vorzugsweise eine voraktivierte Carbonsäure oder ein Carbonsäurehalogenid, ein symmetrisches oder gemischtes Anhydrid oder ein Aktivester, eingesetzt werden. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart) beschrieben. Aktivierte Ester werden zweckmäßig in situ gebildet, z.B. durch Zusatz von HOBt (1-Hydroxybenzotriazol) oder N-Hydroxysuccinimid.

Die Umsetzung erfolgt in der Regel in einem inerten Lösungsmittel, bei Verwendung eines Säurehalogenids in Gegenwart eines säurebindenden Mittels vorzugsweise einer organischen Base wie Triethylamin, Dimethylanilin, Pyridin, Dimethylaminopyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die erfindungsgemäßen Verbindungen der Formel I, I-a, I-b, I-c oder I-d, wie zuvor beschrieben oder als bevorzugt beschrieben, sind oxidationsstabil und zeigen bei Lagerung eine reduzierte Gelbfärbung bis keine Gelbfärbung.

Erfindungsgemäße Verbindungen der Formel I, wie zuvor beschrieben, eignen sich ebenfalls als Antiglykierungsmittel (Antiglycation agents) und wirken der Bildung von AGEs (advanced glycation endproducts) entgegen.

Erfindungsgemäße Verbindungen eignen sich als Kontrastreduktionsmittel, d.h. sie können dunkle Hautstellen gegebenenfalls aufhellen bzw. helle Hautstellen gegebenenfalls abdunkeln.

Ein weiterer Erfindungsgedanke ist ein Verfahren zum Färben einer, insbesondere proteinhaltigen, Matrix, bei dem in einem Färbeschritt direkt durch Einwirken einer Dispersion und/oder Lösung und/oder Emulsion einer Verbindung der Formel I, I-a, I-b, I-c oder I-d, wie zuvor beschrieben, auf die Matrix dieselbe gefärbt wird.

Dabei kann in einem Vorbehandlungsschritt die Matrix zur Beeinflussung und insbesondere zur Verbesserung des Färbeverhaltens derselben mittels eines Vorbehandlungsmittels vorbehandelt werden. Ein solches Vorbehandlungsmittel kann basisch, sauer oder neutral sein, eine oxidative Wirkung, zum Beispiel durch Vorhandensein eines Oxidationsmittels wie Wasserstoffperoxid, aufweisen und gegebenenfalls Wasser enthalten. Üblicherweise wird der Vorbehandlungsschritt vor dem Färbeschritt durchgeführt.

Ein weiterer Gegenstand der Erfindung sind Zubereitungen, die zumindest eine Verbindung der Formel I, I-a, I-b, I-c oder I-d, wie zuvor beschrieben, enthalten.

Bei der Zubereitung handelt es sich dabei üblicherweise um eine topisch anwendbare Zubereitung, beispielsweise um kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe. Handelt e sich um Medizinprodukte, so enthalten die Zubereitungen einen für das Medizinprodukt geeigneten Träger.

Topisch anwendbar bedeutet hier, dass die Zubereitung äußerlich und örtlich angewendet wird, d.h. dass die Zubereitung geeignet sein muss, um beispielsweise auf die Haut aufgetragen werden zu können.

Bevorzugt werden die topischen Zubereitungen als kosmetische oder dermatologische Zubereitung eingesetzt, insbesondere bevorzugt als kosmetische Zubereitung.

Der Begriff Zubereitung wird gleichbedeutend auch als Mittel oder Formulierung verstanden.

Die Zubereitungen können die genannten notwendigen oder optionalen Bestandteile umfassen oder enthalten, daraus im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

In bevorzugten Ausführungsformen wird die mindestens eine Verbindung der Formel I, I-a, I-b, I-c oder I-d mit den definierten oder als bevorzugt angegebenen Substituenten in den erfindungsgemäßen Zubereitungen typischerweise in Mengen von 0,05 bis 10 Gew.-%, bevorzugt in Mengen von 0,1 Gew.-% bis 5 Gew.-% und besonders bevorzugt in Mengen von 0,5 bis 2 Gew.-%, eingesetzt.

Ein weiterer Gegenstand der Erfindung ist ein Verfahren zur Herstellung einer solchen Zubereitung, wie zuvor beschrieben, wobei zumindest eine Verbindung der Formel I, I-a, I-b, I-c oder I-d, wie zuvor beschrieben, mit zumindest einem für kosmetische, pharmazeutische, dermatologische Zubereitungen, Medizinprodukte oder Haushaltsprodukte geeigneten Träger und gegebenenfalls Hilfsstoffen und/oder Füllstoffen vermischt wird, insbesondere dispergiert und/oder emulgiert und/oder gelöst wird. Geeignete Trägerstoffe sowie Aktiv- oder Hilfsstoffe sind im nachfolgenden Teil ausführlich beschrieben.

Bevorzugt ist die Zubereitung als Mehrkomponentensystem ausgestaltet, wobei zumindest eine Verbindung nach Formel I, wie zuvor beschrieben, als Entwicklungskomponente, zumindest eine Kupplungskomponente, optional ein Vorbehandlungsmittel, optional eine weitere Entwicklungskomponente und/oder zumindest ein Oxidationsmittel auf zumindest zwei Zubereitungskomponenten verteilt sind. Bevorzugt weist eine erste Zubereitungskomponente zumindest eine Verbindung nach Formel I und zumindest eine Kupplungskomponente und zumindest einen für kosmetische, pharmazeutische, dermatologische Zubereitungen oder Haushaltsprodukte geeigneten Träger und gegebenenfalls zumindest ein nicht-oxidatives Vorbehandlungsmittel auf, während eine zweite Kupplungskomponente zumindest ein Oxidationsmittel, insbesondere Wasserstoffperoxid aufweist.

Des Weiteren können, um z.B. weitere Farbanpassungen vornehmen zu können, die Verbindungen der Formel I, I-a, I-b, I-c oder I-d mit vorbekannten Oxidationsfarbstoffkomponenten kombiniert werden.

Geeignete Oxidationsfarbstoffkomponenten vom Entwicklertyp sind p-Phenylendiamin und dessen Derivate. Geeignete p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Weitere geeignete p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen. Als weitere geeignete Entwicklerkomponenten können Verbindungen eingesetzt werden, die mindestens zwei aromatische Kerne enthalten, die mit Amino- und/oder Hydroxylgruppen substituiert sind. Weitere geeignete Entwicklerkomponenten werden insbesondere ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetra-methylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Weitere geeignete zweikernige Entwicklerkomponenten werden ausgewählt aus N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es möglich sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidin-Derivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Weitere geeignete Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Als Pyrazolopyrimidine sind insbesondere Pyrazolo[1,5-a]pyrimidine geeignet, wobei bevorzugte Pyrazolo[1,5-a]pyrimidine ausgewählt sind aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-alpyrimidin sowie ihre physiologisch verträglichen Salze und ihre tautomeren Formen.

Weitere geeignete Entwicklerkomponenten werden ausgewählt aus mindestens einer Verbindung aus der Gruppe, die gebildet wird aus p-Phenylendiamin, p-Totuylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1 H-imidazol-1-yl)propyl]amin, N,M-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Weitere geeignete Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,0001 bis 10 Gew.-%, vorzugsweise 0,001 bis 5 Gew.-%, jeweils bezogen auf die anwendungsbereite Zubereitung oder Zubereitungskomponente, verwendet.

Geeignete Oxidationsfarbstoffkomponenten vom Kupplertyp werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetrahydrochinoxalin, und/oder Gemischen aus zwei oder mehreren Verbindungen aus einer oder mehreren dieser Klassen.

Weitere verwendbare Kupplerkomponenten, wie m-Aminophenole bzw. deren Derivate, werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamitio)benzol, 3-Ethylamino-4-methylphenol, 2,4-Dichlor-3-aminophenol und deren physiologisch verträglichen Salzen.

Weitere verwendbare Kupplerkomponeten, wie z.B. 3-Diaminobenzole bzw. deren Derivate, werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl)amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Weitere verwendbare Kupplerkomponeten, wie z.B. o-Diaminobenzole bzw. deren Derivate, werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Weitere verwendbare Kupplerkomponeten, wie z.B. Di- beziehungsweise Trihydroxybenzole und deren Derivate, werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Weitere verwendbare Kupplerkomponeten, wie z.B. Pyridinderivate, werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Als Kupplerkomponente geeignete Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Als Kupplerkomponente geeignete Indolderivate werden ausgewählt aus 4-Hydroxyindol, 6-Hydroxyindol und 7-Hydroxyindol und deren physiologisch verträglichen Salzen.

Als Kupplerkomponente geeignete Indolinderivate werden bevorzugt ausgewählt aus 4-Hydroxyindolin, 6-Hydroxyindolin und 7-Hydroxyindolin und deren physiologisch verträglichen Salzen.

Als Kupplerkomponente geeignete Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Geeignete Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Amino-phenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethylamino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydro-xyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 10 Gew.-%, vorzugsweise 0,001 bis 5 Gew.-%, jeweils bezogen auf die anwendungsbereite Zubereitung oder Zubereitungskomponente, verwendet.

In den beschriebenen Zubereitungen, die erfindungsgemäß mindestens eine Verbindung nach Formel I, I-a, I-b, I-coder I-d enthalten, können weiterhin auch Farbpigmente enthalten sein, wobei der Schichtaufbau der Pigmente nicht limitiert ist.

Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Die Zubereitungen können neben der mindestens einen Verbindung nach Formel I, I-a, I-b, I-c oder I-d sowie den gegebenenfalls anderen Inhaltsstoffen weitere organische UV-Filter, sogenannte hydrophile oder lipophile Sonnenschutzfilter, die im UVA-Bereich und/oder UVB-Bereich und/oder IR und/oder VIS-Bereich (Absorber) wirksam sind, enthalten. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische wie auch anorganische UV Filter sind in den Patentanmeldungen EP-A 0 487 404 sowie WO2009/077356 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vertrieben unter dem Namen "Escalol 507" von der Fa. ISP, Glyceryl PABA, PEG-25 PABA, z. B. vertrieben unter dem Namen "Uvinul P25" von der Fa. BASF.

Salicylate: Homosalate vertrieben unter dem Namen "Eusolex HMS" von der Fa. Merck; Ethylhexyl salicylate, z. B. vertrieben unter dem Namen "Neo Heliopan OS" von der Fa. Symrise, Dipropylene glycol salicylate, z. B. vertrieben unter dem Namen "Dipsal" von der Fa. Scher, TEA salicylate, z. B. vertrieben unter dem Namen "Neo Heliopan TS" von der Fa. Symrise.

β,β-Diphenylacrylate Derivate: Octocrylene, z. B. vertrieben unter dem Namen "Eusolex® OCR" von der Firma Merck, "Uvinul N539" von der Fa. BASF, Etocrylene, z. B. vertrieben unter dem Namen "Uvinul N35" von der BASF. Ferner z.B. Methoxycrylen, vertrieben unter dem Namen Solastay S1 von der Firma Hallstar.

Benzophenone Derivate: Benzophenone-1, z. B. vertrieben unter dem Namen "Uvinul 400"; Benzophenone-2, z. B. vertrieben unter dem Namen "Uvinul D50" ; Benzophenone-3 oder Oxybenzone, z. B. vertrieben unter dem Namen "Uvinul M40";Benzophenone-4, z. B. vertrieben unter dem Namen "Uvinul MS40"; Benzophenone-9, z. B. vertrieben unter dem Namen "Uvinul DS-49" von der Fa. BASF, Benzophenone-5, Benzophenone-6, z. B. vertrieben unter dem Namen "Helisorb 11" von der Fa. Norquay, Benzophenone-8, z. B. vertrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vertrieben unter dem Namen "Mexoryl SD" von der Fa. Chimex, 4-Methylbenzylidenecamphor, z. B. vertrieben unter dem Namen "Eusolex 6300" von der Fa. Merck, Benzylidenecamphorsulfonsäure, z. B. vertrieben unter dem Namen "Mexoryl SL" von der Fa. Chimex, Camphor benzalkonium methosulfate, z. B. vertrieben unter dem Namen "Mexoryl SO" von der Fa. Chimex, Terephthalylidenedicamphorsulfonsäure, z. B. vertrieben unter dem Namen "Mexoryl SX" von der Fa Chimex, Polyacrylamidomethylbenzylidenecamphor vertrieben unter dem Namen "Mexoryl SW" von der Fa. Chimex.

Phenylbenzimidazol Derivate: Phenylbenzimidazolesulfonsäure, z. B. vertrieben unter dem Namen "Eusolex 232" von der Fa. Merck, Dinatrium phenyl dibenzimidazole tetrasulfonat, z. B. vertrieben unter dem Namen "Neo Heliopan AP" von der Fa. Symrise.

Phenylbenzotriazol Derivate: Drometrizole trisiloxane, z. B. vertrieben unter dem Namen "Silatrizole" von der Fa. Rhodia Chimie, Methylenebis(benzotriazolyl)tetramethylbutylphenol in fester Form, z. B. vertrieben unter dem Namen "MIXXIM BB/100" von der Fa. Fairmount Chemical, oder in mikronisierter Form als wässrige Dispersion, z. B. vertrieben unter dem Namen "Tinosorb M" von der Fa. Ciba Specialty Chemicals.

Triazin Derivate: Ethylhexyltriazone, z. B. vertrieben unter dem Namen "Uvinul T150" von der Fa. BASF, Diethylhexylbutamidotriazone, z. B. vertrieben unter dem Namen "Uvasorb HEB" von der Fa. Sigma 3V. Weitere Triazinderivate sind exemplarisch 2,4,6-tris(diisobutyl 4'-aminobenzalmalonate)-s-triazine, oder 2,4,6-Tris-(biphenyl)-1,3,5-triazine, Butyl 4-({4-{[4-(butoxycarbonyl)phenyl]amino}-6-[(3-{1,3,3,3-tetramethyl-1-[(trimethylsilyl)oxy]disiloxanyl} propyl)amino]-1,3,5-triazin-2-yl}amino)benzoate, vertrieben unter dem namen Mexoryl SBS. Struktur von Mexoryl SBS: 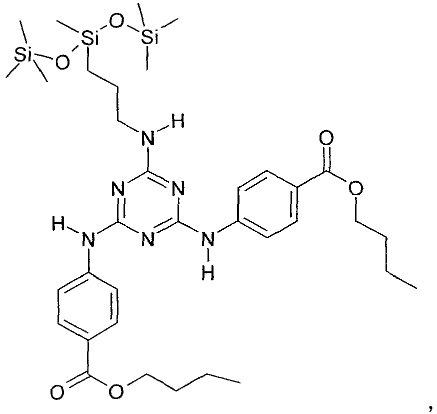 sowie Bis-ethylhexyloxyphenol methoxyphenyl triazine, z.B. vertrieben unter dem Namen Tinosorb S durch die Firma BASF.

Anthranilin Derivate: Menthyl anthranilate, z. B. vertrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vertrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vertrieben unter dem Namen Uvasorb K2A von der Fa. Sigma 3V und Mischungen dieses enthaltend.

Piperazinderivate wie beispielsweise die Verbindung 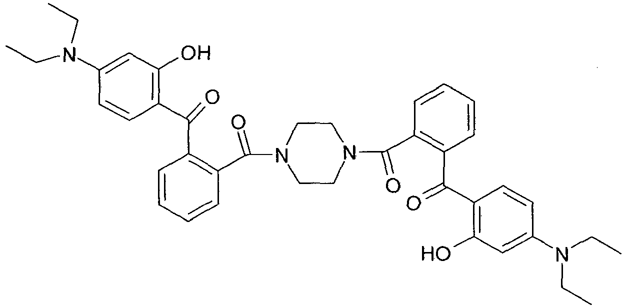 oder die UV-Filter der folgenden Strukturen 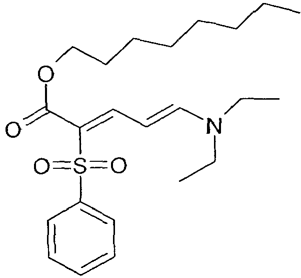 oder

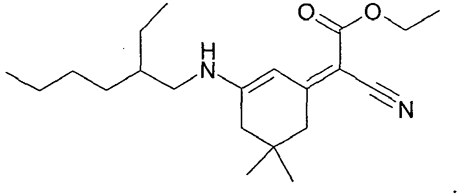

Bevorzugt kann auch mit UV-Filtern auf Basis von Polysiloxancopolymeren mit einer statistischen Verteilung gemäß nachfolgender Formel kombiniert werden, wobei z.B. a = 1,2; b= 58 und c=2,8 sind:

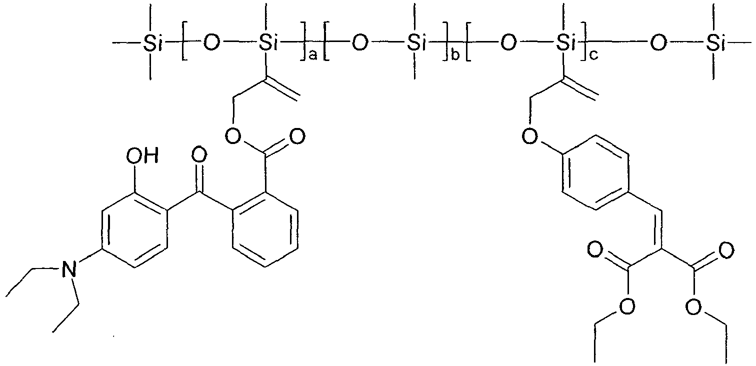

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate,

Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethyipropyi)-4,4-diphenylbutadiene, 2,4-Bis[5-1 (dimethylpropyl)benzoxazol-2-yl(4-phenyl)imino)-6-(2-ethylhexyl)imino-1,3,5-triazine und Mischungen davon.

Diese organischen UV-Filter werden in der Regel in einer Menge von 0,01 Gewichtsprozenten bis 20 Gewichtsprozenten, vorzugsweise 1 Gew.-% - 10 Gew.-%, in Formulierungen eingearbeitet.

Die Zubereitungen können neben den Verbindungen der Formel I sowie den gegebenenfalls anderen organischen UV-Filtern, wie zuvor beschrieben, weitere anorganische UV-Filter, sogenannte partikuläre UV-Filter enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Aminosäuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt einzusetzende partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbehandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination einzusetzenden behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben,
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2Sl3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   ∘ ZnO nachbehandelt mit Polymethylhydrogenosiloxane,
   ∘ Nanogard Zinc Oxide FN der Fa. Nanophase Technologies
   ∘ "SPD-Z1" der Fa Shin-Etsu (ZnO nachbehandelt mit einem Silikongepfropften Acrylpolymer, dispergiert in Cyclodimethylsiloxane
   ∘ "Escalol Z100" der Fa ISP (Aluminiumoxid nachbehandeltes ZnO dispergiert in einer ethylhexyl methoxycinnamate/PVPhexadecene/methicone copolymer Mischung)
   ∘ "Fuji ZNO-SMS-10" der Fa. Fuji Pigment (ZnO nachbehandelt mit Siliciumdioxid und Polymethylsilesquioxan);
   ∘ Unbehandeltes Ceroxide Mikropigment z.B. mit der Bezeichnung "Colloidal Cerium Oxide" der Fa Rhone Poulenc
   ∘ Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbehandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B. das Produkt UV-Titan M261 der Fa. Sachtleben in Kombination mit dem erfindungsgemäßen UV-Schutzmittel verwendet werden.

Diese anorganischen UV-Filter werden in der Regel in einer Menge von 0,1 Gewichtsprozenten bis 25 Gewichtsprozenten, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Daher kann es bevorzugt sein, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Bevorzugte Zubereitungen können auch mindestens einen weiteren kosmetischen Wirkstoff enthalten, beispielsweise ausgewählt aus Antioxidantien, Anti-aging-Wirkstoffen, Anti-Cellulite Wirkstoffen, Selbstbräunungssubstanzen, hautaufhellenden Wirkstoffen oder Vitaminen.

Ferner sind erfindungsgemäße Farbstoffe mit allen Wirkstoffen und Hilfstoffen kombinierbar, wie sie in WO2009/098139 systematisch gelistet sind. Insbesondere gehören diese Stoffe zu den darin aufgeführten Verwendungskategorien "Moisturizers and Humectants", "Desquamating agents", "Agents for improving the barrier function", "Depigmenting Agents", "Antioxidants", "Dermo-relaxing or dermo-decontracting agents", "Antiglycation agents", "Agents for stimulating the synthesis of dermal and/or epidermal macromolecules and/or for preventing their degradation", "Agents for stimulating fibroblast or keratinocyte proliferation and/or keratinocyte differentiation", "Agents for promoting the maturation of the horny envelope", "NO-synthase inhibitors", "Peripheral benzodiazepine receptor (PBR) antagonists", "Agents for increasing the activity of the sebaceous glands", "Agents for stimulating the energy metabolism of cells", "Tensioning agents", "Fat-restructuring agents", "Sliming agents", "Agents for promoting the cutaneous microcirculation", "Calmatives or anti-irritants", "Sebo-regulating or anti-seborrhoic agents", "Astringents", "Cicatrizing agents", "Anti-inflammatory agents", "Antiacne agents".

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Geeignete Antioxidantien sind auch Verbindungen der Formeln A oder B 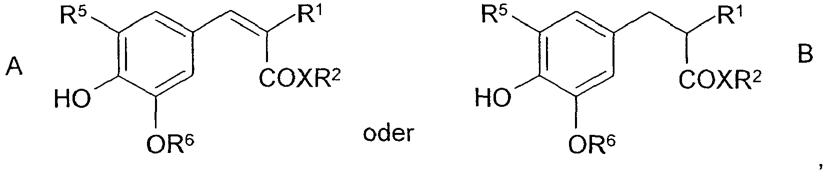 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils unabhängig voneinander H oder lineares oder verzweigtes Alkyl mit 1 bis 8 C-Atomen,
R⁵ H oder lineares oder verzweigtes Alkyl mit 1 bis 8 C-Atomen oder lineares oder verzweigtes Alkoxy mit 1 bis 8 C-Atomen und R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex^{®} ST Liquid) und/oder 2-(4-Hydroxy-3,5-dimethoxybenzyl)-malonsäure-bis-(2-ethylhexyl)ester (z.B. RonaCare^{®} AP).

Ferner ist die Kombination mit 2-(4-Hydroxy-3-methoxybenzyliden)-malonsäure-bis-isopropylester bzw. 2-(4-Hydroxy-3-methoxybenzyl)-malonsäure-bis-isopropylester (hydrogenated diisopropyl vanilidene malonate) bevorzugt. Analoges gilt für entsprechende Bis-ethylester.

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin-Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myoinositol-phosphat (DIP), cyclisches 2,3-diphosphoglycerat (cDPG), 1,1-Diglycerin-Phosphat (DGP), ß-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-aging Wirkstoffe Produkte der Firma Merck wie z.B. 5,7-Dihydroxy-2-methyl-chromon, vermarktet unter dem Handelsnamen RonaCare®Luremine oder die Handelsprodukte Ronacare®Isoquercetin, Ronacare®Tilirosid oder Ronacare®Cyclopeptide-5 verwendet werden.

Ferner können die erfindungsgemäßen Zubereitungen mindestens einen Selbstbräuner als weiteren Inhaltsstoff enthalten.

Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Die Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin, Niacinamide, Azelainsäure, Elaginsäure, Tranexamsäure, Kalium 4-Methoxysalicylat, Maulbeerbaumextract, Magnesium-ascorbyl-phosphat, Süßholzwurzelextrakt, Emblica, Ascorbinsäure oder Rucinol sowie Substanzen wie unter WO2007121845 beschrieben.

Die Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein. Für eine innerliche Anwendung sind Darreichungsformeln wie Kapseln, Dragees, Pulver, Tabletten-Lösungen oder Lösungen geeignet.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.

Bevorzugte Hilfsstoffe stammen aus der Gruppe der Konservierungsstoffe, Stabilisatoren, Lösungsvermittler, Färbemittel, Geruchsverbesserer. Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Die erfindungsgemäße Mischung kann in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide).

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate ausfolgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)-glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsformen der Erfindung sind in den Beispielen dargestellt und werden in der nachfolgenden Beschreibung näher erläutert, ohne den Umfang der vorliegenden Erfindung zu beschränken.

### Beispiele:

### Beispiel 1: Darstellung von 4-Phenylazo-bezoesäure-2-methly-4-oxo-4H-pyran-3-yl-ester

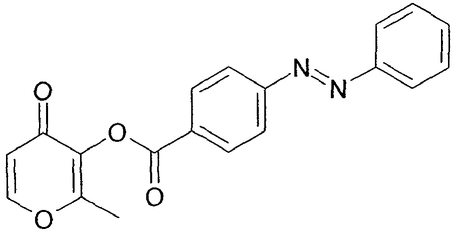

5,20g (23,0 mmol) 4-(Phenylazo)-benzoesäure und 3,50g (27,8mmol) 3-Hydroxy-2-methyl-4-pyranon werden zusammen mit 0,28g (2,30 mmol) 4-(Dimethylamino)-pyridin in 50 mL THF suspendiert. Zur roten Suspension wird eine Lösung von 5,70g (27,6 mmol) N, N'-Dicyclohexylcarbodiimid in 10 mL THF getropft. Dabei wird ein Temperaturanstieg von 22°C auf 30°C beobachtet. Die Reaktionsmischung dickt ein. Es wird 48h bei Raumtemperatur (RT) nachgerührt. Dann werden 2,50g (27,8 mmol) Oxalsäure-dihydrat zugegeben und 2,5h bei RT nachgerührt. Nach Abkühlen auf 0°C wird filtriert und der Nutschkuchen mit 50mL THF nachgewaschen. Der Nutschkuchen wird mit 150mL THF ausgerührt und erneut abgesaugt. Die vereinigten Waschlösungen werden auf ca. 150mL eingeengt. Daraus werden über Nacht bei RT rote Kristalle erhalten, die noch mit etwas Essigester nachgewaschen und dann i. Vak. getrocknet werden. Auswaage: 2,25g. (29% d. Th.)
¹H-NMR (400MHz, DMSO-d₆): δ= 2.36 (s, 3H), 6.54 (d, ³*J* = 5.7Hz, 1H), 7.68 (m, 3H), 8.00 (m, 2H), 8.10 (m, 2H), 8.27 (d, ³*J* = 5.8Hz, 1H), 8.35 (m, 2H).

### Beispiel 2: Darstellung von 4-(4-Dimethylamino-phenylazo)-benzoesäure-2-methyl-4-oxo-4H-pyran-yl-ester

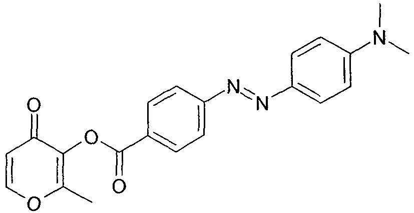

Zu einer Suspension von 3,90g (30,9 mmol) 3-Hydroxy-2-methyl-4-pyranon und 7,00g (26,0 mmol) 4-Dimethylaminobenzol-4-carbonsäure in 50 mL THF werden 0,32g (2,60 mmol) 4-(Dimethylamino)-pyridin hinzugefügt. Innerhalb von 6 min wird eine Lösung von 6,40g (31,0 mmol) Dicyclohexylcarbodiimid in 10 mL THF zugetropft. Die rot braune Suspension erwärmt sich auf 28°C. Es wird 48h nachgerührt und dann werden 4,00g (44.4 mmol) Oxalsäuredihydrat zugefügt. Nach 2 h Nachrühren bei RT wird die Reaktionsmischung auf 0°C abgekühlt, filtriert und der Rückstand mit 70mL THF nachgewaschen. Das Filtrat wird mit 200mL Essigester und 100mL Wasser versetzt, extrahiert und die Phasen getrennt. Die organische Phase wird noch mal mit 55mL Wasser gewaschen, über Natriumsulfat getrocknet, filtriert und im Vakuum eingeengt. Dann wird aus Isopropanol umkristallisiert. Nach Absaugen wird der Rückstand mit 150mL THF gerührt und der Feststoff abgesaugt, danach der Feststoff nochmals aus 100mL Acetonitril und bei Bedarf aus Isopropanol umkristallisiert. Es werden insgesamt 280mg erhalten.
¹H-NMR(400MHz, DMSO-d₆): δ= 2.34 (s, 3H), 3.20 (s, 6H), 6.53 (d, 1H), 6.88 (m, 2H), 7.84 (m, 2H), 7.95 (m,2H), 8.24 (m, 3H).

### Beispiel 3:

### Darstellung von 3-{4-[(E)-2-(4-Dimethylamino-phenyl)-vinyl]-pyridin-1-yl}-propionsäure-2-methyl-4-oxo-4H-pyran-3-yl ester

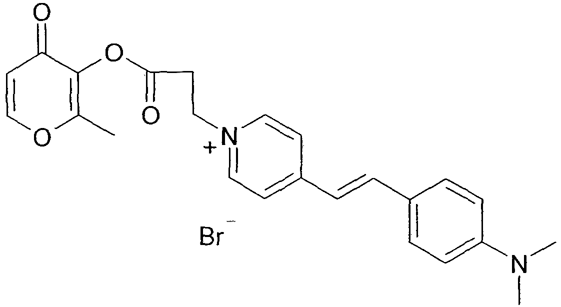

1,00g (2,65mmol) E-1-(2-Carboxyethyl)-4-(4-(dimethylamino)styryl)pyridinium bromid, 0,40g (3,18 mmol) 3-Hydroxy-2-methyl-4-pyranon und 0,03g (0,27 mmol) 4-(Dimethylamino)pyridin werden in 7mL THF unter Schutzgasatmosphäre suspendiert. Zur roten Suspension werden 0,66g (3,18mmol) N,N'-Dicyclohexylcarbodiimid, gelöst in 3mL THF, bei RT zugetropft und über Nacht nachgerührt. Danach werden 0,29g (3,18 mmol) Oxalsäure-Dihydrat hinzugefügt und 72 Stunden nachgerührt. Dann werden 15mL THF zugegeben und es wird 1 Stunde nachgerührt. Es wird im Vakuum filtriert und der Rückstand mit 10mL THF nachgewaschen. Die Mutterlauge wird im Vakuum einrotiert und der erhaltene rote Feststoff wird mit 15mL Essigester versetzt. Es wird im Vakuum filtriert und mit Essigester nachgewaschen. Es werden 750mg Feststoff erhalten, der an 15g Kieselgel mit 10mL MeOH eluiert wird. Nach Entfernen des Lösemittels werden 80mg roter amorpher Feststoff (R_{f} = 0.07; MeOH) erhalten.

### Beispiel 4:

### Synthese von 6-[6-(3-Dimethylamino-propylamino)-1,3-dioxo-1 H,3H-benzo[de]isoquinolin-2-yl]-hexanoic acid 2-methyl-4-oxo-4H-pyran-3-yl ester (4)

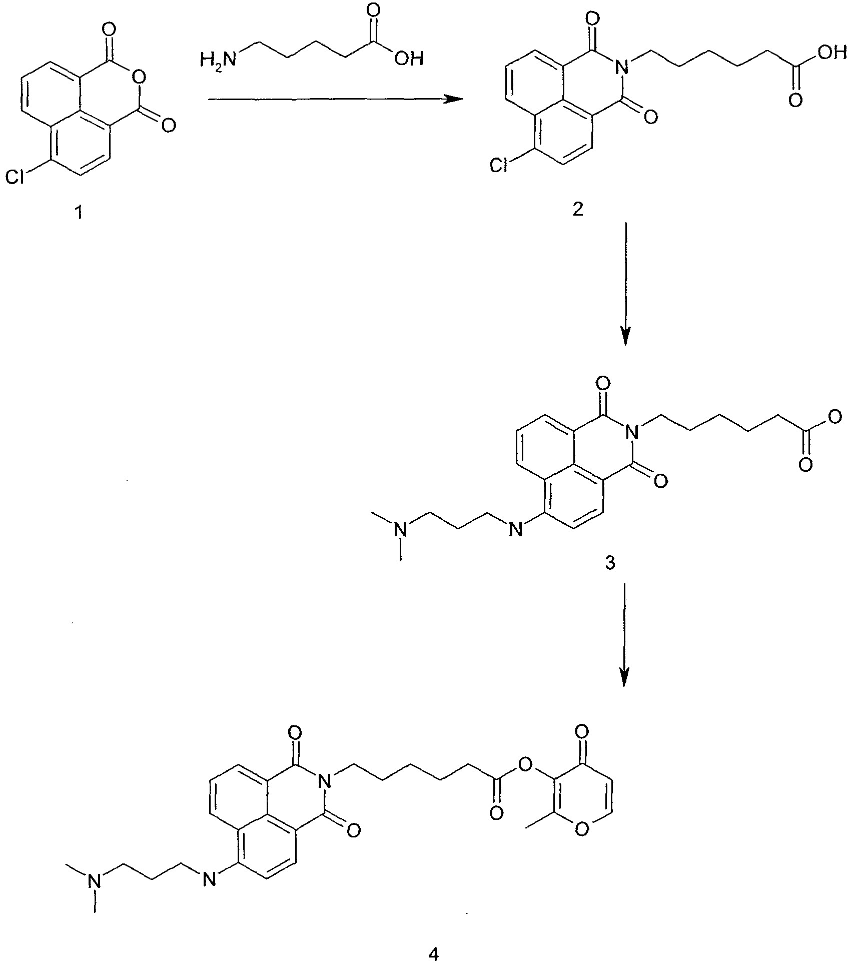

Die Verbindung 3 wird hergestellt, indem kommerziell erhältliches 6-Chlorobenzo[de]isochromene-1,3-dion gemäß W. Adam et al, Tetrahedron: Asymmetry 2003, 14(10), 1355-1361 zur Verbindung 2 umgesetzt wird, die entsprechend nach der in US 5235045 A beschriebenen Methode zur Verbindung 3 aminiert wird. Die Umsetzung der freien Säure der Verbindung 3 mit 3-Hydroxy-2-methyl-4-pyranon erfolgt analog zu Beispiel 1.

### Beispiel 5: Darstellung von 4-Phenylazo-benzoesäure- 1,2-dimethyl-4-oxo-1,4-dihydro-pyridin-3-yl ester

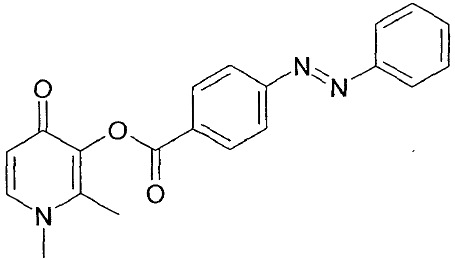

4-Phenylazo-benzoesäure-1,2-dimethyl-4-oxo-1,4-dihydro-pyridin-3-yl ester wird in Analogie zu Beispiel 1 hergestellt, wobei anstelle 3-Hydroxy-2-methyl-4-pyranon das 3-Hydroxy-1,2-dimethyl-1H-pyridin-4-on als Edukt Verwendung findet.

### Beispiel 6: Nachweis der Proteinaffinität im Lysinmodell:

Die Substanzen aus Beispiel 1 und 2 werden jeweils zu 10mg in einen 10 mL Meßkolben eingewogen und in 5 mL Tetrahydrofuran (THF) gelöst. Der Meßkolben wird nun mit einer Lysinlösung in destilliertem Wasser bis zur Marke aufgefüllt. Die Konzentration der Lysinlösung beträgt 200mg/L Wasser. Man rührt die Lösung im Meßkolben für 24 h bei 35°C. Nach 1 h und nach 24 h werden Proben gezogen. Davon pipettiert man je 5 µL mit einer Mikropipette auf die Startzone einer HPTLC Silica gel 60 F245 Platte (Merck Art. 1.05628). Zur Entwicklung der HPTLC Platte verwendet man das Laufmittel bestehend aus THF/Heptan im Verhältnis 75/25 enthaltend 0,1 vol% Ameisensäure. Nach Entwicklung detektiert man in einem CAMAG ATS4 bei 254nm und 366nm und ermittelt die Rf-Werte.

Ergebnis: Substanz 1 besitzt einen Rf-Wert von 0,62, Substanz 2 von 0,57. Bereits nach 1 h in Kombination mit Lysin sind beide Substanzen deutlich, nach 24h komplett, in einer Proteinreaktion mit Lysin umgesetzt. Die Ausgangssubstanzen sind nicht mehr detektierbar. Demgegenüber sind Maillardprodukte entstanden, die in der Startzone der HPTLC-Platte detektiert werden.

### Beispiel 7: Textilfärbung

Die Substanzen gemäß Beispiel 1 und Beispiel 2 werden zu je 5% in Wasser gelöst/dispergiert. Der pH-Wert des Wassers ist alkalisch. Die wässrige Lösung/Dispersion wird zunächst aufgekocht und anchließend auf unter 40°C langsam abgekühlt, bevor man die Wolle hinzugibt (ca. 1 kg Wolle auf 25-30 Liter Wasser). Nach 24h wird die wolle herausgenommen und mit Leitungswasser gut gespült.

### Beispiel A: Haarfarbe aus verschiedenen Komponenten:

### Konponente A:

Tocopherol, Linalool, Geraniol, Disodium EDTA, Parfum, ascorbic acid, alcohol denat., Sodium sulfite, Sodium hydroxide, Sodium cocoyl isethionate, Bis-ethylhexyl Hydroxydimethoxy Benzylmalonate, Sodium lauryl sulfate, Ammonia, Lanolin alcohol, Glycol distearate, Sodium laureth sulfate, Glyceryl stearate, Ceteary alcohol, Aqua.

### Komponente B:

Aqua, hydrogen peroxide, cetearyl alcohol, PPG-38-buteth-37, petrolatum, laureth-2, sodium cetearyl sulfate, salicylic acid, disodium phosphate, phosphoric acid, etidronic acid.

### Komponente C:

Ethanolische Lösung der Farbstoffe gemäß Beispiel 2 und/oder 3 (je 2 Gew.-%) zudem enthaltend Bis-ethylhexyl Hydroxydimethoxy Benzylmalonate (1 Gew.-%).

### Komponente D:

Ethanolische Lösung von Bis-ethylhexyl Hydroxydimethoxy Benzylmalonate (1 Gew.-%).

### Anwendung:

Zur Haarfärbung wird bevorzugt in folgender Reihenfolge vorgegangen: Zunächst wird das Haar mit Komponente C vorbehandelt, Anschließend werden die Komponenten B und C gemischt und auf das Haar appliziert. Nach erfolgter Färbung wird Komponente D appliziert.

### Beispiel B: Haarfarbe aus verschiedenen Komponenten:

### Konponente A:

Tocopherol, Linalool, Geraniol, Disodium EDTA, Parfum, Toluene-2,5-diamine sulfate, ascorbic acid, alcohol denat., Sodium sulfite, Sodium hydroxide, Sodium cocoyl isethionate, Bis-ethylhexyl Hydroxydimethoxy Benzylmalonate, 2-Methylresorcinol, 6-Amino-m-cresol, 4-Amino-2-hydroxy-toluol, 4-Amino-m-cresol, Sodium lauryl sulfate, Ammonia, Lanolin alcohol, Glycol distearate, Sodium laureth sulfate, Glyceryl stearate, Ceteary alcohol, Aqua.

### Komponente B:

Aqua, hydrogen peroxide, cetearyl alcohol, PPG-38-buteth-37, petrolatum, laureth-2, sodium cetearyl sulfate, salicylic acid, disodium phosphate, phosphoric acid, etidronic acid.

### Komponente C:

Ethanolische Lösung des Farbstoffs nach Beispiel 1 und /oder des Beispiels 2, 3 oder 4 (je 2 Gew.-%) zudem enthaltend Bis-ethylhexyl Hydroxydimethoxy Benzylmalonate (1 Gew.-%).

### Komponente D:

Ethanolische Lösung von Bis-ethylhexyl Hydroxydimethoxy Benzylmalonate (1 Gew.-%).

### Anwendung:

Zur Haarfärbung wird bevorzugt in folgender Reihenfolge vorgegangen: Zunächst wird das Haar mit Komponente C vorbehandelt, Anschließend werden die Komponenten B und C gemischt und auf das Haar appliziert. Nach erfolgter Färbung wird Komponente D appliziert.

Für alle Beispielrezepturen der Zubereitungen gilt, dass diese optional auch frei von UV-Filtern hergestellt werden können.

**Beispiel C: W/O Emulsion**

| | **a** | b | c | d | e |
|---|---|---|---|---|---|
| Cetyl PEG/PPG-10/1 dimethicone (Abil EM 90) | 3,00 | 3,00 | 3,00 | 3,00 | 3,00 |
| Polyglyceryl-4 isostearate (Isolan Gl 34) | 1,50 | 1,50 | 1,50 | 1,50 | 1,50 |
| Butylphthalimide isopropylphthalimide (Pelemol^{®} BIP) | 5,00 | 5,00 | 5,00 | 5,00 | 5,00 |
| Dimethyl isosorbide (Arlasolve DMI) | 5,00 | 5,00 | 5,00 | 5,00 | 5,00 |
| Farbstoff nach Beispiel 1 | 2,00 | | | | 1,00 |
| Farbstoff nach Beispiel 2 | | 2,00 | | | 1,00 |
| Farbstoff nach Beispiel 3 | | | 2,00 | | 1,00 |
| Farbstoff nach Beispiel 4 | | | | 2,00 | 1,00 |
| Uvinul^{®} A Plus (DHHB) | | 1,00 | 1,00 | 1,00 | |
| Ascorbinsäure | | | 0,37 | 1,00 | 3,00 |
| Mineral Oil | 8,00 | 8,00 | 8,00 | 8,00 | 8,00 |
| Ethylhexyl stearate (Tegosoft^{®} OS) | 5,00 | 5,00 | 5,00 | 5,00 | 5,00 |
| Cyclomethicone (and) Aluminium/Magnesiu m Hydroxide Stearate (Gilugel SIL 5) | 5,00 | 5,00 | 5,00 | 5,00 | 5,00 |
| Preservative | 1,00 | 1,00 | 1,00 | 1,00 | 1,00 |
| Water | Ad 100 | Ad 100 | Ad 100 | Ad 100 | Ad 100 |
| NaCl | 0,50 | 0,50 | 0,50 | 0,50 | 0,50 |
| EDTA | 0,10 | 0,10 | 0,10 | 0,10 | 0,10 |
| Citronensäure q.S. | | | | | |

| | | | | | |
|---|---|---|---|---|---|
| Herstellung: Pelemol^{®} BIP, Arlasolv DMI und Emulgatoren werden vorgelegt. Die Farbstoffe der Beispiele 1 bis 4 und Uvinul^{®} A Plus werden darin gelöst. Die restlichen Bestandteile der Ölphase werden zugegeben und homogen vermischt. Unter Rühren wird die mit Citronensäure auf pH=4-5 eingestellte Wasserphase einemulgiert. Anschließend wird homogenisiert. Die Emulsionen können unter schonenden Bedingungen bei Raumtemperatur hergestellt werden. | | | | | |

**Beispiel D: Wasserfestes Sonnenschutzspray mit Auftragskontrolle**

| A | | | |
|---|---|---|---|
| Farbstoff nach Beispiel 1, 2, 3 oder 4 | 1,00 | 1,00 | 2,00 |
| Diethylhexyl Syringylidenemalonate, Caprylic/Capric Triglyceride (Oxynex^{®} ST Liquid) | | 0,50 | |
| RonaCare^{®} AP | | 2,00 | |
| Ascorbyl Palmitate | | | 1,00 |
| Cyprylic/capric Triglyceride (Miglyol 812 N) | 7,00 | 7,00 | 7,00 |
| Butylphthalimide isopropylphthalimide (Pelemol^{®} BIP) | 9,00 | 9,00 | 9,00 |
| C12-15 alkyl benzoate (Tegosoft^{®} TN) | 10,00 | 10,00 | 10,00 |
| Phenethyl benzoate (X-Tend 226) | 5,00 | 5,00 | 5,00 |
| RonaCare^{®} Tocopherolacetat | 1,00 | 1,00 | 1,00 |

| B | | | |
|---|---|---|---|
| Cyclopentasiloxane (Dow Corninq 245) | 43,80 | 41,30 | 41,80 |
| Phenyltrimethicone (Dow Corninq 556) | 2,00 | 2,00 | 2,00 |
| Cyclopentasiloxane, dimethiconol Dow Corninq 1501 Fluid | 20,00 | 20,00 | 20,00 |
| Parfümöl (q.s.) | 0,20 | 0,20 | 0,20 |

| | | | |
|---|---|---|---|
| Herstellung: Die Komponenten der Phase A werden bei Raumtemperatur zusammengefügt und gerührt bis eine klare Lösung oder homogene Disperison vorliegt. Anschließend wird Phase B gemischt und unter Rühren zu Phase B gegeben. Man rührt weiter, bis letztlich das homogene Produkt vorliegt. Zu Zugabe von Antioxidantien wie Oxynex^{®} ST Liquid, RonaCare^{®} AP oder Ascorbylpalmitat kann die Stabilität der erfindungsgemäßen Substanzen erhöht werden. | | | |

**Beispiel E: Pump Haartönungsspray**

| A | | | |
|---|---|---|---|
| Farbstoff nach Beispiel 1 | 1,00 | 1,00 | 4,00 |
| Farbstoff nach Beispiel 2 | 1,00 | | |
| Farbstoff nach Beispiel 3 | | 1,00 | |
| Farbstoff nach Beispiel 4 | | | 1,00 |
| Ethanol 96% reinst | Ad 100 | Ad 100 | Ad 100 |
| PVPNA copolymer PVP/VA W 735 | 6,00 | 6,00 | 6,00 |

| B | | | |
|---|---|---|---|
| Diethylhexyl Syringylidenemalonate, Caprylic/Capric Triglyceride (Oxynex^{®} ST Liquid) | 0,06 | 0,25 | 0,50 |
| PEG-75 Lanolin BHT (Solan E - Low Dioxane) | 0,20 | 0,20 | 0,20 |
| Parfum (Frag 280853 Green Activating) | 0,10 | 0,10 | 0,10 |

| C | | | |
|---|---|---|---|
| Wasser, demineralisiert | 13,00 | 13,00 | 13,00 |
| Titriplex III | 0,10 | 0,10 | 0,10 |
| PEG-12 dimethicone Dow Corning 193 Fluid | 0,50 | 0,50 | 0,50 |
| 0,1% D&C Red No 33 (Cl 17200) in Wasser | 0,20 | 0,20 | 0,20 |
| PEG-40 Hydrogenated Castor Oil (Cremophor RH 410) | 1,00 | 1,00 | 1,00 |

| | | | |
|---|---|---|---|
| Herstellung: Phase A vorlösen, bis eine klare Lösung vorliegt. Unter Rühren Phase B zu Phase A geben. Phase C vormischen und zum Rest geben, rühren, bis eine homogene Mischung entstanden ist. | | | |

**Beispiel F: W/O-Emulsionen**

| **Emulsion** | **A** | **B** | **C** | **D** | **E** | **F** |
|---|---|---|---|---|---|---|
| Polyglyceryl-2-Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | | 0,3 | | 1 |
| Glycin | 0,3 | 0,3 | 0,5 | 0,4 | | |
| Alkohol | | 5 | 2 | 5 | 4 | |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid | 5 | 6 | 4 | | | 4 |
| Zinkoxid | 5 | | | | | |
| Bis-Ethylhexyloxyphenol Methoxyphenyltriazin | | 3 | 3 | 2 | | |
| Ethylhexyltriazon | | 4,5 | 3 | | 3 | |
| Farbstoff A | 1,0 | | 1,5 | 1,0 | 3,0 | |
| Diethylhexylbutamidotriazon | | | 1,5 | 4 | | |
| Butyl Methoxydibenzoylmethan | 2 | 3 | 4 | | 1 | 3 |
| Uvinul^{®} A Plus | | | | 4 | 2 | |
| Ethylhexylmethoxycinnamat | | | | | 7 | 5 |
| Farbstoff nach Beispiel 2 | | 4,0 | 0,5 | 1,5 | | 0,5 |
| Taurin | 0,1 | | | 0,5 | 0,2 | |
| Vitamin E Acetat | 0,2 | 02 | | 0,3 | 0,1 | 0,5 |
| Na₂H₂EDTA | 0,1 | 0,1 | 0,2 | 0,2 | 0,2 | 0,5 |
| C8-C16 Alkylpolyglycosid | 1 | | | | | |
| Parfüm, Konservierungsmittel | q.s. | q.s | q.s | q.s | qs. | qs. |
| Farbstoffe, usw. | q.s. | q.s. | q.s. | q.s | q.s. | q.s. |
| Natriumhydroxid | q.s. | q.s. | q.s. | q.s | q.s. | q.s. |
| Wasser | ad 100,0 | ad 100,0 | ad 100,0 | ad 100,0 | ad 100,0 | ad 100,0 |

**Beispiel G: Haarpflegeformulierung**

| **Gehalt in g Komponente per 100 g Formulierung** | | | | | | |
|---|---|---|---|---|---|---|
| **Komponente** | **A** | **B** | **C** | **D** | **E** | **F** |
| Disodium EDTA | 0.100 | 0.100 | 0.100 | 0.100 | 0.100 | 0.100 |
| Oxynex^{®}ST | 2.000 | 2.000 | 2.000 | 2.000 | 2.000 | 2.000 |
| Farbstoff nach Beispiel 1 | 0,10 | 0,25 | 0,50 | 1,50 | 2,00 | 4,00 |
| Farbstoff nach Beispiel 2 | 0,50 | 1,00 | 1,50 | 2,00 | 2,50 | 3,00 |
| Hexamidine düsethionate | 0.100 | 0 | 0 | 0 | 0 | 0 |
| Tetrahydrocurcumin | 0 | 0.500 | 0 | 0 | 0 | 0 |
| Glycyrrhetinic acid | 0 | 0 | 0.300 | 0 | 0 | 0 |
| Thiotaine®¹ | 0 | 0 | 0 | 5.000 | 0 | 0 |
| N-undecylenoyl-L-phenylalanine | 0 | 0 | 0 | 0 | 1.000 | 0 |
| N-acetyl glucosamine | 0 | 0 | 0 | 0 | 0 | 2.000 |
| Niacinamide | 5.000 | 5.000 | 5.000 | 5.000 | 5.000 | 5.000 |
| Citric acid | 0.015 | 0 | 0 | 0 | 0 | 0 |
| Isohexadecane | 3.000 | 3.000 | 3.000 | 3.000 | 3.000 | 3.000 |
| Isopropyl isostearate | 1.330 | 1.330 | 1.330 | 1.330 | 1.330 | 1.330 |
| Isopropyl N-laurosylsarcosinate | 0 | 0 | 5.000 | 0 | 0 | 0 |
| Sucrose polycottonseedate | 0.670 | 0.670 | 0.670 | 0.670 | 0.670 | 0.670 |
| Polymethylsilsesquioxane | 0.250 | 0.250 | 0.250 | 0.250 | 0.250 | 0.250 |
| Cetearyl glucoside + cetearyl alcohol | 0.200 | 0.200 | 0.200 | 0.200 | 0.200 | 0.200 |
| Behenyl alcohol | 0.400 | 0.400 | 0.400 | 0.400 | 0.400 | 0.400 |
| Ethylparaben | 0.200 | 0.200 | 0.200 | 0.200 | 0.200 | 0.200 |
| Propylparaben | 0.100 | 0.100 | 0.100 | 0.100 | 0.100 | 0.100 |
| Cetyl alcohol | 0.320 | 0.320 | 0.320 | 0.320 | 0.320 | 0.320 |
| Stearyl alcohol | 0.480 | 0.480 | 0.480 | 0.480 | 0.480 | 0.480 |
| Tocopheryl acetate | 0.500 | 0.500 | 0.500 | 0.500 | 0.500 | 0.500 |
| PEG-100 stearate | 0.100 | 0.100 | 0.100 | 0.100 | 0.100 | 0.100 |
| Glycerin | 7.000 | 7.000 | 7.000 | 7.000 | 7.000 | 7.000 |
| Titanium dioxide | 0.604 | 0.604 | 0.604 | 0.604 | 0.604 | 0.604 |
| Polyacrylamide + C13-14 | 3.000 | 2.000 | 2.000 | 2.000 | 2.000 | 2.000 |
| isoparaffin + laureth-7 | | | | | | |
| Panthenol | 1.000 | 1.000 | 1.000 | 1.000 | 1.000 | 1.000 |
| Benzyl alcohol | 0.400 | 0.400 | 0.400 | 0.400 | 0.400 | 0.400 |
| Dimethicone + dimethiconol | 2.000 | 2.000 | 2.000 | 2.000 | 2.000 | 2.000 |
| Water (to 100 g) | to 100 | to 100 | to 100 | to 100 | to 100 | to 100 |
| TOTAL | 100 | 100 | 100 | 100 | 100 | 100 |

**Beispiel H: Haarpflegeformulierung**

| **Gehalt in g Komponente per 100 g Formulierung** | | | |
|---|---|---|---|
| **Komponente** | **G** | **H** | **I** |
| Disodium EDTA | 0.100 | 0.100 | 0.100 |
| Oxynex^{®} ST | 2.000 | 2.000 | 2.000 |
| Farbstoff nach Beispiel 1, 2, 3 oder 4 oder Farbstoffkombination aus den Farbstoffen nach Beispiel 1, 2, 3 und/oder4 | 0.20 | 1.500 | 0,75 |
| Cetyl pyridinium chloride | 0.200 | 0 | 0 |
| Pitera^{®} | 0 | 10 | 0 |
| Ascorbyl glycoside | 0 | 0 | 2.000 |
| Niacinamide | 3.500 | 5.000 | 4.000 |
| Polyquaternium 37 | 0 | 0 | 0 |
| Isohexadecane | 3.000 | 2.500 | 2.000 |
| Isopropyl isostearate | 1.330 | 1.330 | 1.330 |
| Sucrose polycottonseedate | 0.670 | 0.670 | 0.670 |
| Polymethylsilsesquioxane | 0.250 | 0.250 | 0.250 |
| Cetearyl glucoside + cetearyl alcohol | 0.200 | 0.200 | 0.200 |
| Behenyl alcohol | 0.400 | 0.400 | 0.400 |
| Ethylparaben | 0.200 | 0.200 | 0.200 |
| Propylparaben | 0.100 | 0.100 | 0.100 |
| Cetyl alcohol | 0.320 | 0.320 | 0.320 |
| Stearyl alcohol | 0.480 | 0.480 | 0.480 |
| Tocopheryl acetate | 0.500 | 0.500 | 0.500 |
| PEG-100 stearate | 0.100 | 0.100 | 0.100 |
| Glycerin | 7.000 | 7.000 | 7.000 |
| titanium dioxide | 0.604 | 0.604 | 0.604 |
| Polyacrylamide + C13-14 isoparaffin + laureth-7 | 2.000 | 2.000 | 2.000 |
| Panthenol | 1.000 | 1.000 | 1.000 |
| Benzyl alcohol | 0.400 | 0.400 | 0.400 |
| Dimethicone + dimethiconol | 2.000 | 2.000 | 2.000 |
| Water (to 100 g) | to 100 | to 100 | to 100 |
| TOTAL | 100 | 100 | 100 |

**Beispiel I: O/W-Emulsionen**

| **Emulsion** | **A** | **B** | **C** | **D** | **E** | **F** |
|---|---|---|---|---|---|---|
| Glyceryl Stearat Citrat | 2,5 | 2 | 3 | | | |
| Sorbitanstearat | 0,5 | | | 2 | 1,5 | 2 |
| Polyglyceryl-3 Methylglycose Distearat | | | | 2,5 | 3 | 3 |
| Polyglyceryl-2 | | 0,8 | | | | 0,5 |
| Dipolyhydroxystearat | | | | | | |
| Cetearylalkohol | | | | 1 | | |
| Stearylalkohol | 2 | | | | | 2 |
| Cetylalkohol | | 1 | | | 3 | |
| Acrylates/C₁₀₋₃₀ Alkyl Acrylat Crosspolymer | | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-C38 Säuretriglyceride | 0,5 | | 1 | | 1 | |
| Titandioxid | 5 | | | 2 | | |
| 2,2'-Methylen-bis-(6-(2H-benzotriazol-2-yl)-('1,1,3,3-tetramethylbutyl)phenol) | 2,5 | | | | | |
| 2,4,6-Tris-(biphenyl)-1,3,5-triazin | | 2 | | | | |
| Merocyanin an Gelatine gekoppelt | 6 | | 6 | | 10 | 3 |
| Benzotriazol an Gelatine gekoppelt | | 5 | | 10 | | 3 |
| C8-C16 Alkylpolyglycosid | 1 | 0,6 | | | | |
| UVASorb^{®} K2A | | | 2 | | | |
| Uvinul^{®} A Plus | 2 | | | | | 1 |
| Homosalat | | 5 | | 1 | | |
| Phenylbenzimidazol Sulfonsäure | | | 2 | | | 1 |
| Benzophenon-3 | *0,5* | | | | 1 | |
| Octylsalicylat | 5 | 5 | | 2 | | |
| Octocrylen | 2 | | | | 3 | 1 |
| Farbstoff nach Beispiel 1 | 0,1 | 0,2 | 0,3 | 0,4 | 0,5 | 1,0 |
| Farbstoff nach Beispiel 2 | 0,1 | 0,2 | 0,3 | 0,4 | 0,5 | 1,0 |
| Bis-Ethylhexyloxyphenol Methoxyphenyltriazin | | 3 | 2 | 1 | | |
| Parsol^{®} SLX | | | 3 | | | |
| Dihydroxyacetat | | | | | 4 | |
| Taurin | 0,1 | | | 0,5 | 0,2 | |
| 8-Hexadecen-1,16-dicarbonsäure | | 0,2 | | | | |
| Vitamin E Acetat | 0,2 | 0,2 | | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100, 0 |

**Beispiel J: O/W-Emulsionen**

| **Emulsion** | **G** | **H** | **I** | **K** | **L** | **M** |
|---|---|---|---|---|---|---|
| Ceteareth-20 | 1 | 1,5 | 1 | | | |
| Sorbitanstearat | | | 0,5 | | 0,5 | |
| Glyceryl Stearat SE | | | | 1 | 1 | 1,5 |
| Emulgade F^{®} | | | | 2,5 | 2,5 | 3 |
| Cetearylalkohol | | | | 1 | | |
| Stearylalkohol | | | | | 1,5 | |
| Cetylalkohol | | | 0,5 | | | 2 |
| Acrylates/C₁₀₋₃₀ Alkyl Acrylat Crosspolymer | 0,2 | 0,4 | 0,3 | 0,1 | | |
| Carbomer | | | | | 0,3 | |
| Xanthan Gum | | | | 0,4 | | 0,4 |
| C₁₂₋₁₅ Alkyl Benzoat | 5 | 3 | | | 5 | |
| 2-Phenylbenzoat | | 2 | | | | |
| Butylenglycol Dicaprylat/Dicaprat | 5 | | | | 3 | 2 |
| Dicaprylyl Ether | | | | | 2 | |
| Diethylhexylnaphthalat | 2 | | | | | |
| Dicaprylcaprat | | 2 | | | 2 | 2 |
| Cyclomethicon | 5 | | 5 | 10 | | |
| Isohexadecan | | | | 5 | | |
| Mineralöl | | 1 | | | | |
| Propylenglykol | | | 4 | | | |
| Glycerin | 5 | 7 | 3 | 5 | 6 | 8 |
| C18-38 Säuretriglyceride | 0,5 | | 1 | | 1 | |
| Titandioxid | 5 | | 3 | 2 | | |
| Phenylbenzimidazol Sulfonsäure | 1 | | | 1 | 2 | 1 |
| Parsol® SLX | 0,5 | 1,0 | 1,5 | 2,0 | 2,5 | 3,0 |
| Farbstoff nach Beispiel 1 oder 2 | 0,5 | 1,0 | 3,0 | 0,5 | | |
| Farbstoff nach Beispiel 3 | | | 0,5 | 3,0 | 1,0 | 0,5 |
| Kreatinin | 0,1 | 0,01 | 0,05 | | | |
| Kreatin | 0,5 | 0,2 | 0,1 | | | |
| Licorice Extrakt/Licochalkon | | | | 0,5 | | |
| Vitamin E Acetat | 0,2 | | | 0,5 | 0,5 | 0,5 |
| Tapioka Stärke | | 3 | | | 2 | |
| Na₂H₂EDTA | 0,1 | | 0,2 | | | 0,5 |
| Parfüm, Konservierungsmittel | q.s. | q.s. | q.s. | q.s. | q.s. | q.s. |
| Pigmentfarbstoffe | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 900,0 |

**Beispiel K: O/W-Emulsionen zur Hautfärbung mit UV-Schutz**

| **Emulsion** | **N** | **O** | **P** | **Q** | **R** | **S** |
|---|---|---|---|---|---|---|
| Glycerylstearat SE | | 2 | | 2 | | |
| Glycerylstearat | 2 | | 2 | | | |
| PEG-40 Stearat | | | 2 | | 1 | |
| PEG-10 Stearat | | | | 2,5 | 1 | |
| Ceteareth-20 | | | | | | 2,6 |
| Natrium Cetyl Phosphate | | | | | 2 | |
| Glyceryl Stearat, Ceteareth-12, Ceteareth-20, Cetearyl Alcohol, Cetyl Palmitat | | | | | | 5,4 |
| Stearinsäure | 3 | 2 | | | 2 | |
| Stearylalkohol | | 2 | 2 | | | |
| Stearylalkohol | 0,5 | | 2 | | | |
| Cetylalkohol | 3 | | | 2 | | |
| Acrylates/C₁₀₋₃₀ Alkyl Acrylat Crosspolymer | | | 0,2 | | 0,4 | |
| Carbomer | | 0,3 | | 0,3 | 0,3 | |
| Xanthan Gum | | 0,3 | 0,4 | | | |
| C₁₂₋₁₅ Alkyl Benzoat | 5 | | | | 5 | 3 |
| 2-Phenylbenzoat | 5 | | | | | |
| Butylenglycol Dicaprylat/Dicaprat | | 5 | | 4 | | 3 |
| Dicaprylyl Ether | | 2 | | | 3 | |
| Diethylhexylnaphthalat | 3 | | | | | |
| Cyclomethicon | 2 | | 10 | 2 | | |
| Isohexadecan | | | | 2 | 3 | |
| Mineralöl | | | | | 3 | |
| Propandiol | | 3 | | 5 | | |
| Glycerin | 3 | 5 | 10 | 7 | 4 | 5 |
| Titandioxid | 2 | 4 | | | | |
| Zinkoxid | | | | | 2 | |
| Drometrizole Trisiloxane | | | | | 3 | |
| Ethylhexylmethoxycinnamat | | 6 | 5 | | | |
| Phenylbenzimidazol Sulfonsäure | | 0,5 | 2 | | 1 | |
| Homosalat | 5 | | | 7 | | |
| Butyl Methoxydibenzoylmethan | | 3 | | | | |
| Bis-Ethylhexyloxyphenol Methoxyphenyltriazin | | 2 | 3 | | | |
| Octylsalicylat | | | | 5 | | |
| Octocrylen | | | | | 3 | |
| Farbstoff nach Beispiel 1 | 0,25 | 0,5 | 0,75 | 1,0 | 1,25 | 1,5 |
| Parsol^{®} SLX | 4 | | | | | 5 |
| PVP Hexadecen Copolymer | 0,5 | | 1 | | 0,8 | |
| Coenzym Q 10 | 0,2 | 0,02 | | 0,3 | | |
| Vitamin E Acetat | 0,2 | | 0,3 | | 0,8 | 0,5 |
| Na₂H₂EDTA | 0,1 | | | | | 0,5 |
| Parfüm, Konservierungsmittel | q.s. | q.s. | q.s. | q.s. | q.s. | q.s. |
| Pigmentfarbstoffe | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**Beispiel L: wässrige und wässrig/alkoholische Formulierungen**

| | **A** | **E** | **C** | **D** | **E** | **F** |
|---|---|---|---|---|---|---|
| Ethanol | 50 | 5 | 2 | 40 | 15 | |
| Hydroxyethylcellulose | 0.5 | | | | | |
| Acrylates/C10-30 Alkyl Acrylate Crosspolymer | | | | 0,3 | 0,6 | |
| Cocoatnidopropylbetain | | | 0,3 | | | |
| UVASorb® K2A | | | | | 2 | |
| Uvinul® APlus | 5 | | | | | |
| Butyl Methoxydibenzoylmethan | 0,5 | | | 3 | | |
| Dinatrium Phenyl Dibenzimidazol Tetrasulfonat | | 2 | 1 | | | |
| Phenylbenzimidazol Sulfonsäure | | 5 | 3 | | 2 | 4 |
| Farbstoff nach Beispiel 1 | 0,1 | 0,25 | 0,5 | 1 | 2 | 3 |
| Farbstoff nach Beispiel 2 | 3 | 2 | 1 | 0,5 | 0,25 | 0,1 |
| Farbstoff nach Beispiel 3 | 0,1 | 0,25 | 0,5 | 1 | 2 | 3 |
| Farbstoff nach Beispiel 4 | 3 | 2 | 1 | 0,5 | 0,25 | 0,1 |
| Farbstoff nach Beispiel 5 | 0,5 | 0,5 | 0,5 | 0,5 | 0,5 | 0,5 |
| C₁₂₋₁₅ Alkyl Benzoat | | | | 3 | | |
| C18-36 Triglycerid Fettsäure | | | | 1 | | |
| Butylenglycol Dicaprylat/Dicaprat | 2 | | | | | |
| C12-13 Alkyl Tartrat | | | | | 5 | |
| Cyclomethicon | 4 | | | 2 | | |
| Insekt Repellent^{®} 3535 | | | | 5 | | |
| Dimethicon | | | | | 3 | |
| PVP Hexadecen Copolymer | | 0,5 | | 1 | | 0.5 |
| Ethylhexyloxyglycerin | | 0,5 | | | | |
| Glycerin | 5 | 7 | 3 | 8 | | S |
| Butylenglycol | | | 5 | | 5 | |
| Metylpropandiol | | | | 4 | | |
| Vitamin E Acetat | | 0,3 | 0,2 | 0,5 | | |
| Panthenol | 0.5 | | 0,2 | | | 0,3 |
| Kreatinin | | | 0,01 | | 0,02 | |
| Creatin | | | 0,1 | | 0,2 | |
| PEG-40 Hydriertes Ricinusöl | | 0,5 | 0,3 | | | 0.5 |
| Trinatrium EDTA | 0,3 | 0,2 | 0,2 | 0,2 | 0,2 | 0,5 |
| Konservierungsmittel | q.s. | q.s. | q.s. | q.s. | q.s. | q.s. |
| Natriumhydroxid | q.s. | q.s. | q.s. | q.s. | q.s. | q.s. |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**Beispiel M: kosmetische Schäume**

| **Emulsion** | **A** | **B** | **C** |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20 Stearat | 3 | | |
| Sorbitanstearat | | 0,8 | |
| C₁₂₋₁₅ Alkyl Benzoat | 5 | | |
| C₁₂₋₁₃ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | | |
| Propylenglykol | | | 5 |
| Glycerin | 5 | 7 | |
| UVASorb® K2A | | | 2 |
| Uvinul® A Plus | 2 | 3 | |
| Parsol SLX^{®} | | 3 | |
| Farbstoff nach Beispiel 1 | 1,0 | | |
| Farbstoff nach Beispiel 2 | | 2,0 | |
| Farbstoff nach Beispiel 3 | | | 1,5 |
| Farbstoff nach Beispiel 4 | | | 1,5 |
| Farbstoff nach Beispiel 5 | | 1,0 | |
| Octocrylen | 2 | | |
| Bis-Ethylhexyloxyphenol Methoxyphenyltriazin | | 3 | |
| 2,2'-Methylen-bis-(6-(2H-benzotriazol-2-yl)-4-(1,1,3,3-tetramethylbutyl)-phenol) | | | 8 |
| 2,4,6-Tris-(biphenyl)-1,3 5-triazin | 5 | | 4 |
| C8-C16 Alkylpolyglycoside | 1 | | |
| Vitamin E Acetat | 0,6 | 0,5 | 0,2 |
| Kreatin/Kreatinin | | | 0,5 |
| BHT | | | 0,1 |
| Na₂H₂EDTA | 0,50 | | |
| Parfum, Konservierungsmitte | q.s. | q.s. | q.s. |
| Farbstoffe, usw. | q.s. | q.s. | q.s. |
| Natriumhydroxid | q.s. | | q.s. |
| kaliumhydroxid | | q.s. | |
| Wasser | ad 100,0 | ad 100,0 | ad 100,0 |

**Beispiel N: kosmetische Schäume**

| **Emulsion** | **D** | **E** | **F** | **G** |
|---|---|---|---|---|
| Stearinsäure | 2 | | | |
| Palmitinsäure | | | 3 | 3 |
| Cetylalkohol | 2 | 2 | | |
| Cetylstearylalkohol | | | 2 | 2 |
| Stearylalkohol | | | | |
| PEG-100 Stearat | | 4 | | |
| PEG-40 Stearat | 2 | | | |
| PEG-20 Stearat | | | 3 | 3 |
| Sorbitanstearat | 0,8 | | | |
| Tridecyl Trimellitate | | 5 | | |
| C₁₂₋₁₅ Alkyl Benzoat | | | 3 | 3 |
| Butylenglycol | 8 | | | |
| Dicaprylat/Dicaprat | | | | |
| Octyldodecanol | | 2 | | |
| Cocoglyceride | | | | 2 |
| Dicaprylyl Ether | | | 2 | 2 |
| Cyclomethicon | | | | |
| Dimethicon | 1 | | 2 | 2 |
| Isohexadecan | | 3 | | |
| Methylpropandiol | | 4 | | |
| Propylenglykol | | | | |
| Glycerin | 5 | | 6 | 6 |
| NeoHeliopan^{®} AP | | 2 | | |
| Phenylbenzimidazol Sulfonsäure | 1 | | | 1 |
| Farbstoff nach Beispiel 1 | 0,5 | 0,5 | 0,5 | 0,5 |
| Ethylhexylmethoxycinnama t | 5 | | 4 | 4 |
| Ethylhexyltriazon | | 2 | | 1 |
| Eusolex T-AVO^{®} | 2 | | | |
| Diethylhexylbutamidotriazo n | 1 | | | |
| Butyl Methoxydibenzoylmethan | 2,5 | | 2 | 2 |
| Bis-Ethylhexyloxyphenol Methoxy-phenyltriazin | 2 | | | |
| Vitamin E Acetat | 0,2 | | 0,3 | 0,3 |
| Na₂H₂EDTA | | | | |
| Parfum, | | | | |
| Konservierungsmitte | | | | |
| Farbstoffe, usw. | | | | |
| Natriumhydroxid | | q-s. | q.s. | |
| Triethanolamin | q.s. | | | q.s. |
| Wasser | ad 100,0 | ad 100,0 | ad 100,0 | ad 100,0 |

## Patentansprüche

1. Verbindungen der Formel I 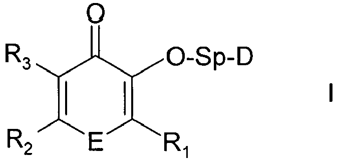 wobei
E NR₄ oder O bedeutet,
Sp eine Bindung, alk, -C(O)- oder -(CO)-alk bedeutet,
R₁, R₂ oder R₃ jeweils unabhängig voneinander -H, -A, -OA, -(CH₂)ₚ-
OH, -C(O)OA, COOH oder COOX bedeuten,
p eine ganze Zahl von 1 bis 4 bedeutet,
X das Gegenion zu der [COO-]-Gruppe ist,
R₄ A bedeutet,
D ein Substituent der Formel II, III, IV, V, VI, VII, VIII, IX, X, XI oder XII ist, 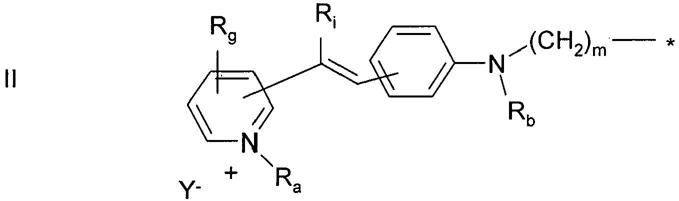 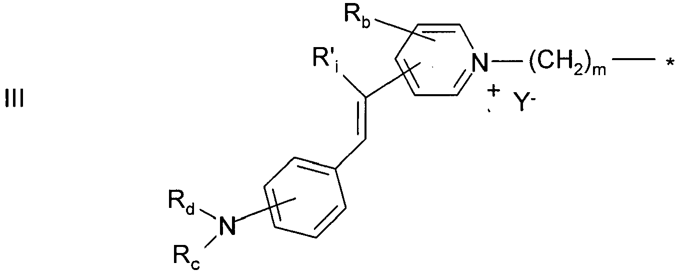 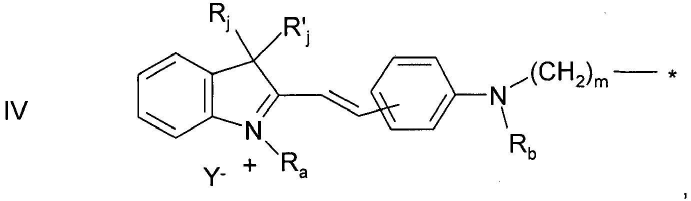 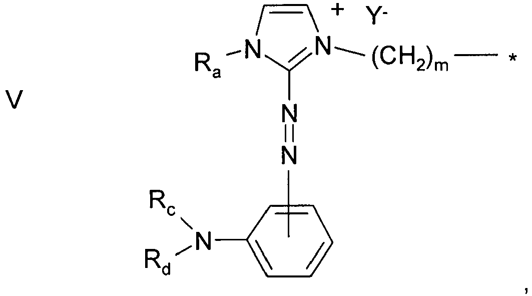 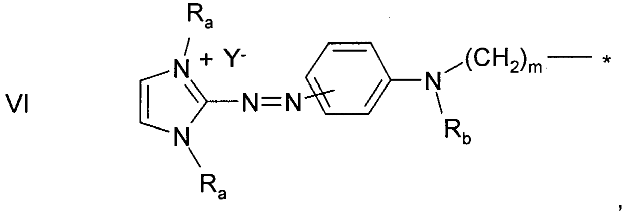 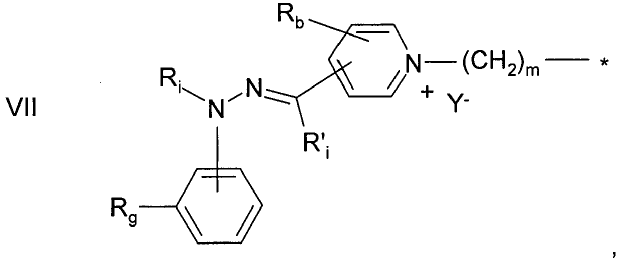 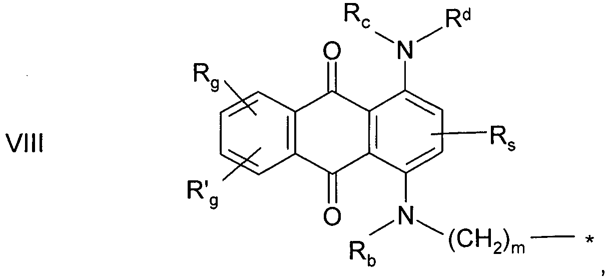 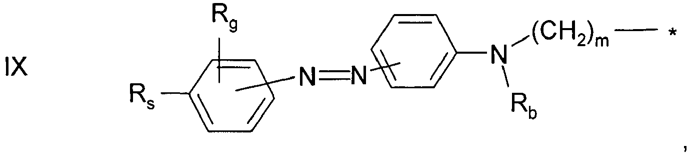 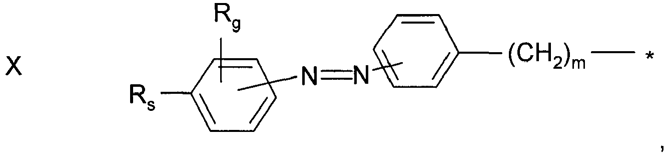 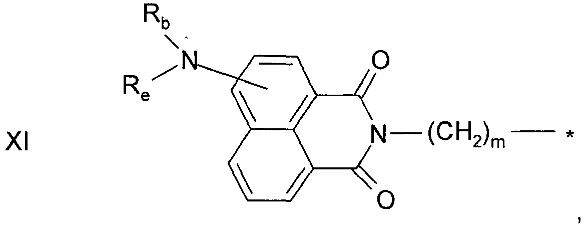 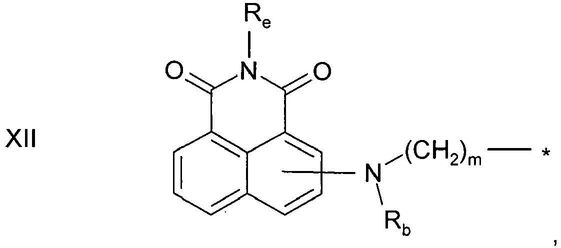 wobei
Rₐ, Rⱼ und R'ⱼ jeweils unabhängig voneinander A bedeuten,
R_{b} H oder A bedeutet,
R_{g} und R'_{g} jeweils unabhängig voneinander H, Hal, NA₂, CN, COOH, OH, CF₃, OA, OC(O)A, C(O)OA, NHC(O)A, NHSO₂A, SO₂NA₂ bedeuten,
Rₛ H, A, NA₂, OA oder SO₃Y bedeutet,
Rᵢ und R'ᵢ jeweils unabhängig voneinander H oder A bedeuten,
R_{c} und R_{d} jeweils unabhängig voneinader H oder A bedeuten, wobei A mit mindestens einer OH-Gruppe substituiert sein kann,
Rₑ eine Alkylgruppe mit 1 bis 6 C-Atomen bedeutet, die durch mindestens eine Gruppe NA₂ oder NA₃Y substituiert ist,
Y ein Anion einer organischen oder anorganischen Säure oder ein Kation ist,
A eine lineare oder verzweigte Alkylgruppe mit 1 bis 20 C-Atomen bedeutet,
Hal F, Cl, Br oder I bedeutet,
m 0, 1, 2, 3, 4 oder 5 bedeutet und
alk eine lineare oder verzweigte oder cyclische Alkylen-Gruppe mit 1 bis 18 C-Atomen bedeutet,
und/oder deren Salze, Tautomere, Stereoisomere und/oder Solvate, einschließlich deren Mischungen in allen Verhältnissen.

2. Verbindungen nach Anspruch 1, **dadurch gekennzeichnet, dass** R₁ A bedeutet.

3. Verbindungen nach Anspruch 1 oder 2, **dadurch gekennzeichnet, dass** R₂ und R₃ H bedeuten.

4. Verbindungen nach einem oder mehreren der Ansprüche 1 bis 3,
**dadurch gekennzeichnet, dass** Sp -C(O)- bedeutet.

5. Verfahren zur Herstellung von Verbindungen der Formel I nach einem oder mehreren der Ansprüche 1 bis 4, **dadurch gekennzeichnet, dass** eine Verbindung der Formel XIII 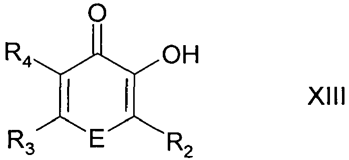 worin R₂, R₃, R₄ und E eine in Anspruch 1, 2, 3 oder 4 angegebene Bedeutung haben,
mit einer Verbindung der Formel XIV
D-Sp-M XIV
umgesetzt wird,
worin D und Sp eine in Anspruch 1, 2, 3 oder 4 angegebene Bedeutung haben, und
M Alkalimetall- oder Erdalkalimetallkation, Halogen, OH oder H bedeutet oder
eine Verbindung der Formel XIII, worin R₂, R₃, R₄ und E eine in Anspruch 1, 2, 3 oder 4 angegebene Bedeutung haben,
mit einem Aktivester der Verbindung der Formel XIV umgesetzt wird, abgeleitet von der freien Säure der Formel XIV, in der M OH und Sp -C(O)- bedeutet, und D eine in Anspruch 1, 2, 3 oder 4 angegebene Bedeutung hat.

6. Zubereitung enthaltend zumindest eine Verbindung der Formel I nach einem oder mehreren der Ansprüche 1 bis 4.

7. Zubereitung nach Anspruch 6, **dadurch gekennzeichnet, dass** es einen kosmetischen, dermatologischen oder pharmakologisch verträglichen Träger enthält.

8. Verfahren zur Herstellung einer Zubereitung nach Anspruch 7, **dadurch gekennzeichnet, dass** mindestens eine Verbindung der Formel I nach einem oder mehreren der Ansprüche 1 bis 4 mit einem Träger und gegebenenfalls mit weiteren Aktiv- oder Hilfsstoffen vermischt wird.

9. Verwendung von Verbindungen der Formel I, nach einem oder mehreren der Ansprüche 1 bis 4 als protein-adhäsive Farbstoffe.

10. Verfahren zum Färben einer proteinhaltigen Matrix, bei dem in einem Färbeschritt direkt durch Einwirken einer Dispersion und/oder Lösung und/oder Emulsion mindestens einer Verbindung der Formel I nach einem oder mehreren der Ansprüche 1 bis 4 auf die Matrix dieselbe gefärbt wird.

## Claims

1. Compounds of the formula I 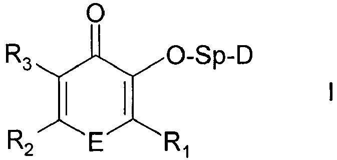 where
E denotes NR₄ or O,
Sp denotes a bond, alk, -C(O)- or -(CO)-alk,
R₁, R₂ or R₃ each, independently of one another, denote -H, -A, -OA, -(CH₂)ₚ-OH, -C(O)OA, COOH or COOX,
p denotes an integer from 1 to 4,
X is the counterion to the [COO-] group,
R₄ denotes A,
D is a substituent of the formula II, III, IV, V, VI, VII, VIII, IX, X, XI or XII, 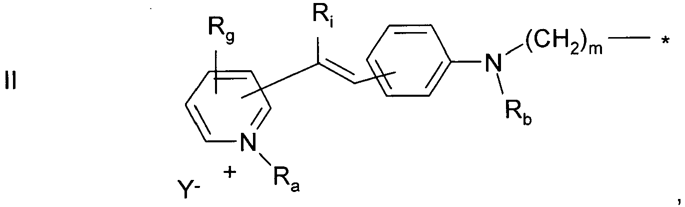 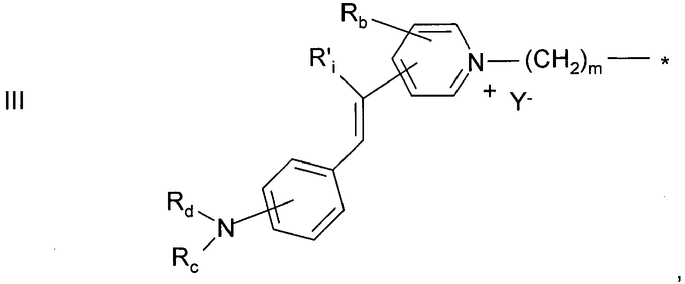 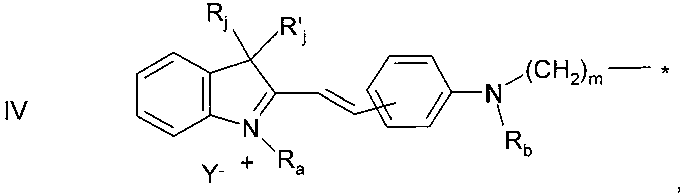 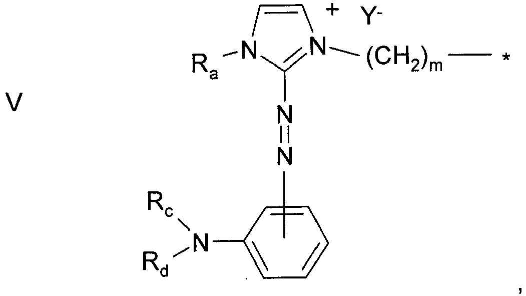 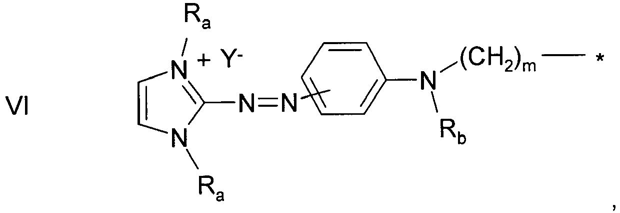 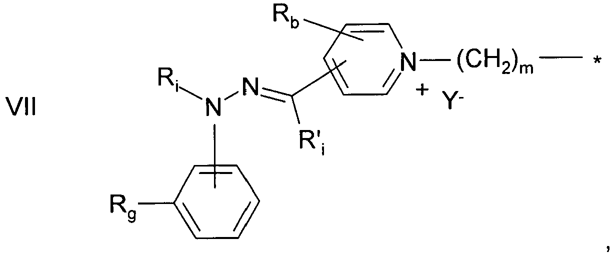 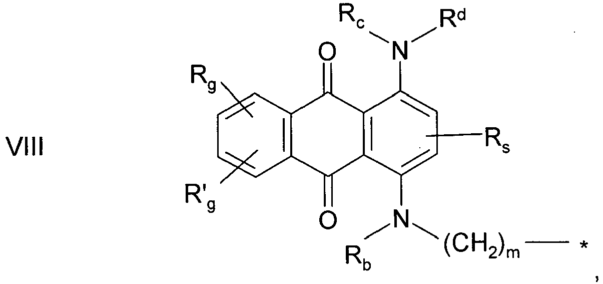 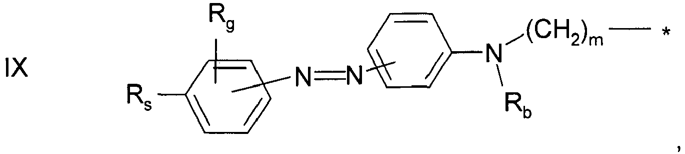 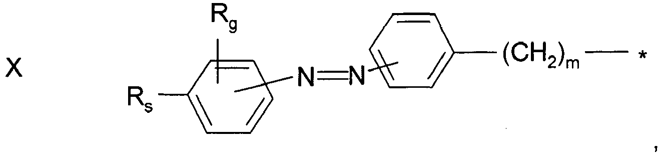 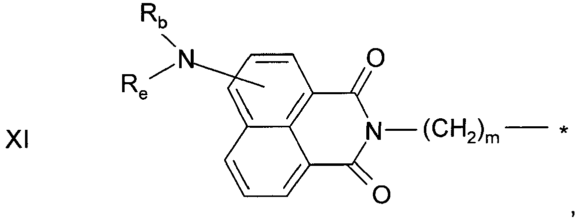 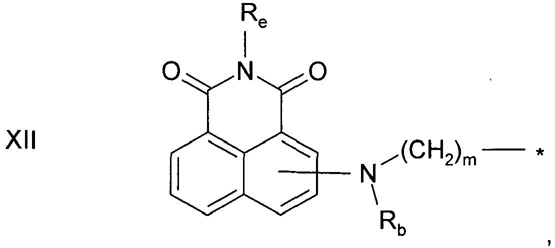 where
Rₐ, Rⱼ and R'ⱼ each, independently of one another, denote A,
R_{b} denotes H or A,
R_{g} and R'_{g} each, independently of one another, denote H, Hal, NA₂, CN, COOH, OH, CF₃, OA, OC(O)A, C(O)OA, NHC(O)A, NHSO₂A, SO₂NA₂,
Rₛ denotes H, A, NA₂, OA or SO₃Y,
Rᵢ and R'ᵢ each, independently of one another, denote H or A,
R_{c} and R_{d} each, independently of one another, denote H or A, where A may be substituted by at least one OH group,
Rₑ denotes an alkyl group having 1 to 6 C atoms which is substituted by at least one group NA₂ or NA₃Y,
Y is an anion of an organic or inorganic acid or a cation,
A denotes a linear or branched alkyl group having 1 to 20 C atoms, Hal denotes F, Cl, Br or I,
m denotes 0, 1, 2, 3, 4 or 5 and
alk denotes a linear or branched or cyclic alkylene group having 1 to 18 C atoms,
and/or salts, tautomers, stereoisomers and/or solvates thereof, including mixtures thereof in all ratios.

2. Compounds according to Claim 1, **characterised in that** R₁ denotes A.

3. Compounds according to Claim 1 or 2, **characterised in that** R₂ and R₃ denote H.

4. Compounds according to one or more of Claims 1 to 3, **characterised in that** Sp denotes -C(O)-.

5. Process for the preparation of compounds of the formula I according to one or more of Claims 1 to 4, **characterised in that** a compound of the formula XIII 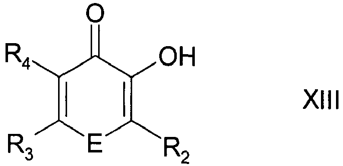 in which R₂, R₃, R₄ and E have a meaning indicated in Claim 1, 2, 3 or 4,
is reacted with a compound of the formula XIV
D-Sp-M XIV,
in which D and Sp have a meaning indicated in Claim 1, 2, 3 or 4, and M denotes an alkali-metal or alkaline-earth metal cation, halogen, OH or H, or
a compound of the formula XIII, in which R₂, R₃, R₄ and E have a meaning indicated in Claim 1, 2, 3 or 4,
is reacted with an active ester of the compound of the formula XIV, derived from the free acid of the formula XIV, in which M denotes OH and Sp denotes -C(O)- and D has a meaning indicated in Claim 1, 2, 3 or 4.

6. Preparation comprising at least one compound of the formula I according to one or more of Claims 1 to 4.

7. Preparation according to Claim 6, **characterised in that** it comprises a cosmetic, dermatological or pharmacologically tolerated vehicle.

8. Process for the preparation of a preparation according to Claim 7, **characterised in that** at least one compound of the formula I according to one or more of Claims 1 to 4 is mixed with a vehicle and optionally with further active substances or assistants.

9. Use of compounds of the formula I according to one or more of Claims 1 to 4 as protein-adhesive dyes.

10. Method for colouring a protein-containing matrix, in which the matrix is coloured directly in one colouring step by the action of a dispersion and/or solution and/or emulsion of at least one compound of the formula I according to one or more of Claims 1 to 4 on the matrix.

## Revendications

1. Composés de formule I 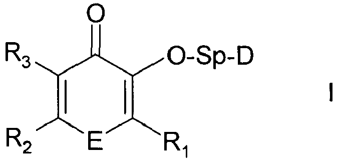 dans lesquels
E désigne NR₄ ou O,
Sp désigne une liaison, alk, -C(O)- ou -(CO)-alk,
R₁, R₂ ou R₃ désignent chacun, indépendamment les uns des autres, -H, -A, -OA,-(CH₂)ₚ-OH, -C(O)OA, COOH ou COOX,
p désigne un nombre entier allant de 1 à 4,
X est le contre-ion du groupement [COO-],
R₄ désigne A,
D est un substituant de formule II, III, IV, V, VI, VII, VIII, IX, X, XI ou XII, 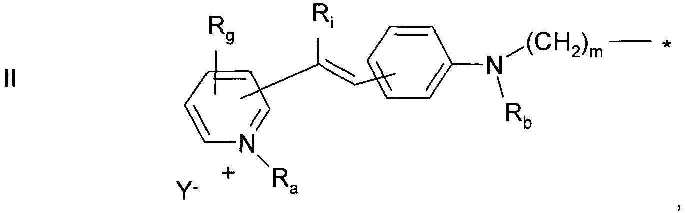 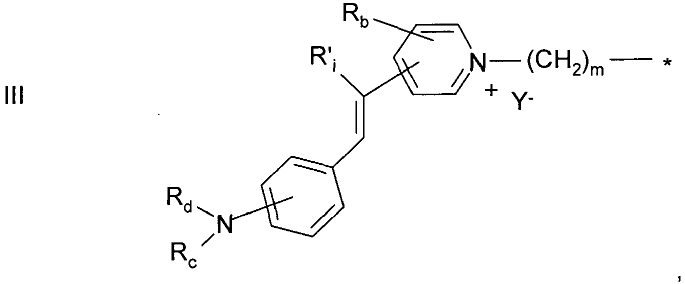 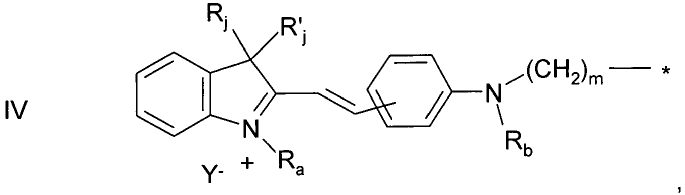 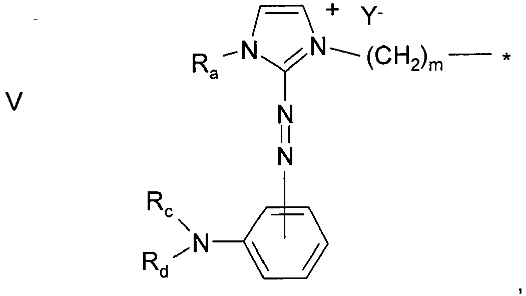 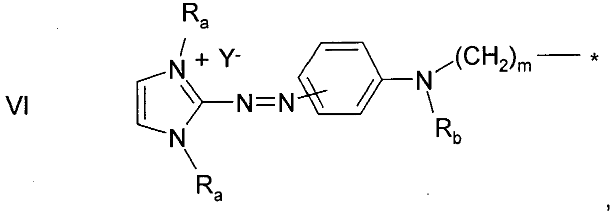 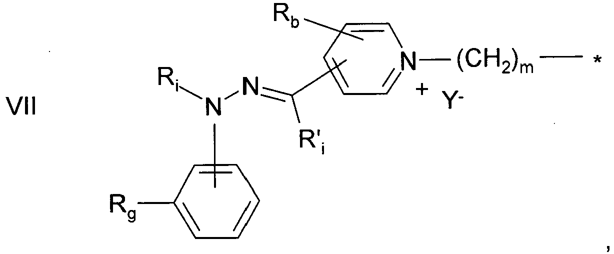 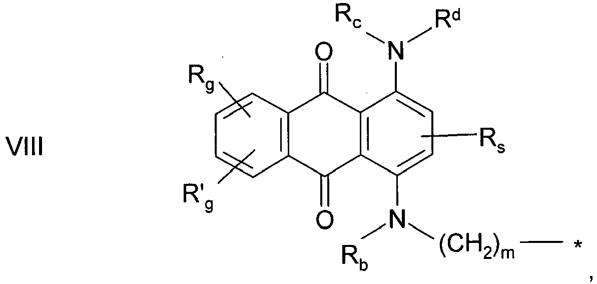 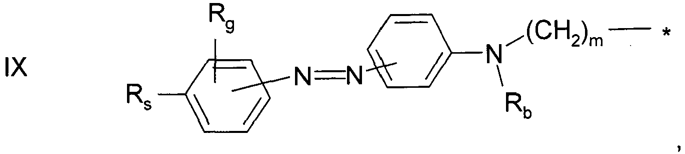 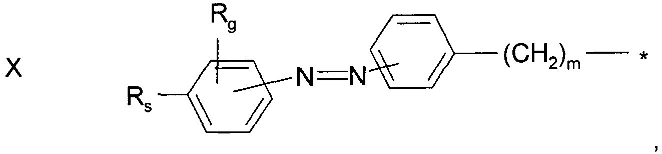 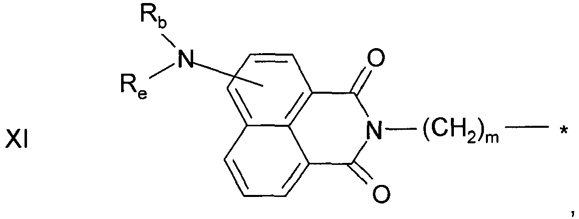 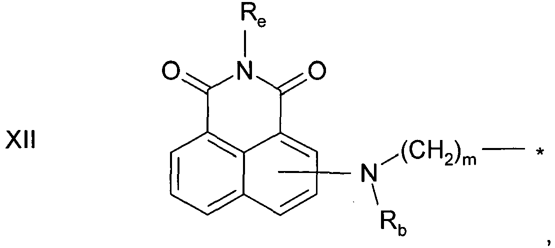 où
Rₐ, Rⱼ et R'ⱼ désignent chacun, indépendamment les uns des autres, A, R_{b} désigne H ou A,
R_{g} et R'_{g} désignent chacun, indépendamment l'un de l'autre, H, Hal, NA₂, CN, COOH, OH, CF₃, OA, OC(O)A, C(O)OA, NHC(O)A, NHSO₂A, SO₂NA₂,
Rₛ désigne H, A, NA₂, OA ou SO₃Y,
Rᵢ et R'ᵢ désignent chacun, indépendamment l'un de l'autre, H ou A,
R_{c} et R_{d} désignent chacun, indépendamment l'un de l'autre, H ou A, où
A peut être substitué par au moins un groupement OH,
Rₑ désigne un groupement alkyle ayant de 1 à 6 atomes de C qui est substitué par au moins un groupement NA₂ ou NA₃Y,
Y est an anion d'un acide organique ou inorganique ou un cation,
A désigne un groupement alkyle linéaire ou ramifié ayant de 1 à 20 atomes de C,
Hal désigne F, Cl, Br ou I,
m désigne 0, 1, 2, 3, 4 ou 5 et
alk désigne un groupement alkylène linéaire ou ramifié ou cyclique ayant de 1 à 18 atomes de C,
et/ou les sels, tautomères, stéréoisomères et/ou solvates de ceux-ci, y compris des mélanges de ceux-ci en toutes proportions.

2. Composés selon la revendication 1, **caractérisés en ce que** R₁ désigne A.

3. Composés selon la revendication 1 ou 2, **caractérisés en ce que** R₂ et R₃ désignent H.

4. Composés selon l'une ou plusieurs parmi les revendications 1 à 3, **caractérisés en ce que** Sp désigne -C(O)-.

5. Procédé de préparation de composés de formule I selon l'une ou plusieurs parmi les revendications 1 à 4, **caractérisé en ce qu'**un composé de formule XIII 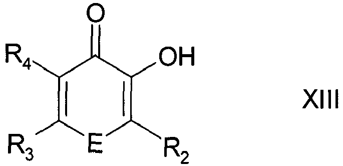 où R₂, R₃, R₄ et E revêtent une signification indiquée selon la revendication 1, 2, 3 ou 4,
est réagi avec un composé de formule XIV
D-Sp-M XIV,
où D et Sp revêtent une signification indiquée selon la revendication 1, 2, 3 ou 4, et
M désigne un cation de métal alcalin ou alcalino-terreux, halogène, OH ou H, ou
un composé de formule XIII, où R₂, R₃, R₄ et E revêtent une signification indiquée selon la revendication 1, 2, 3 ou 4,
est réagi avec un ester actif du composé de formule XIV, dérivé de l'acide libre de formule XIV, où M désigne OH et Sp désigne -C(O)- et D revêt une signification indiquée selon la revendication 1, 2, 3 ou 4.

6. Préparation comprenant au moins un composé de formule I selon l'une ou plusieurs parmi les revendications 1 à 4.

7. Préparation selon la revendication 6, **caractérisée en ce qu'**elle comprend un véhicule cosmétique, dermatologique ou toléré sur le plan pharmacologique.

8. Procédé de préparation d'une préparation selon la revendication 7, **caractérisé en ce qu'**au moins un composé de formule I selon l'une ou plusieurs parmi les revendications 1 à 4 est mélangé avec un véhicule et éventuellement avec d'autres substances actives ou auxiliaires.

9. Utilisation de composés de formule I selon l'une ou plusieurs parmi les revendications 1 à 4, comme colorants adhérant aux protéines.

10. Méthode de coloration d'une matrice contenant des protéines, dans laquelle la matrice est colorée directement en une étape de coloration par l'action d'une dispersion et/ou solution et/ou émulsion d'au moins un composé de formule I selon l'une ou plusieurs parmi les revendications 1 à 4, sur la matrice.
